(19) Europäisches Patentamt
European Patent Office
Office européen des brevets

(11) Publication number: 0 075 436
B1

(12) **EUROPEAN PATENT SPECIFICATION**

(45) Date of publication of patent specification: **02.04.86**

(51) Int. Cl.[4]: **C 07 D 403/10,**
C 07 D 403/12, A 61 K 31/50

(21) Application number: **82304855.8**

(22) Date of filing: **15.09.82**

(54) **Substituted 4,5-dihydro-6-(substituted)-phenyl-3(2H)-pyridazinones and 6-(substituted)phenyl-3(2H)-pyridazinones.**

(30) Priority: **17.09.81 US 302181**
**27.07.82 US 402488**
**13.08.82 US 407973**

(43) Date of publication of application:
**30.03.83 Bulletin 83/13**

(45) Publication of the grant of the patent:
**02.04.86 Bulletin 86/14**

(84) Designated Contracting States:
**AT BE CH DE FR GB IT LI LU NL SE**

(58) References cited:
**EP-A-0 071 060**
**DE-A-2 151 216**
**GB-A-1 383 906**

(73) Proprietor: **WARNER-LAMBERT COMPANY**
**201 Tabor Road**
**Morris Plains New Jersey 07950 (US)**

(72) Inventor: **Sircar, Ila**
**3615 Charter Place**
**Ann Arbor Michigan 48105 (US)**
Inventor: **Bristol, James Arthur**
**1921 High Hollow Drive**
**Am Arbor Michigan 48105 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

The present invention relates to 4,5-dihydro-6-(substituted)phenyl-3(2*H*)-pyridazinone compounds and 6-(substituted)phenyl-3(2*H*)-pyridazinone compounds useful as cardiotonic agents. The present invention also relates to the above mentioned pyridazinone compounds useful as antihypertensive agents.

EP—007546-A1 (A. Natterman & Cie GmbH) published 9th February 1983 discloses compounds of the following formula

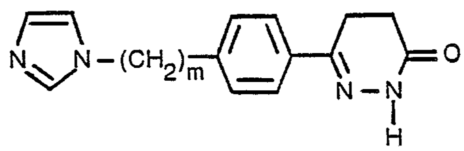

in which m is an integer of from 1 to 12.

SUMMARY OF THE INVENTION

The present invention relates to novel substituted 4,5-dihydro-6-(substituted)phenyl-3(2*H*)-pyridazinone compounds and 6-(substituted)phenyl-3(2*H*)-pyridazinone compounds useful as cardiotonic agents having the structural formula (I):

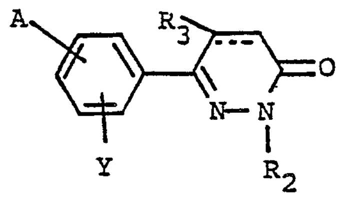

I

wherein ---- represents a double or single bond between two carbon atoms; $R_2$ and $R_3$ are independently hydrogen or $C_{1-6}$ alkyl; Y is H, halogen, $C_{1-6}$ alkyl, and $C_{1-6}$ alkoxy, and A is any of the groups from a—e: and is attached to the 3- or 4-position of the phenyl ring

a. A =

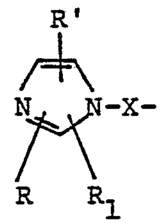

as in the structural formula II wherein $R_1$, R′, and R are independently hydrogen or $C_{1-6}$ alkyl, $CH_2OH$, $SCH_3$, $SOCH_3$, $SO_2CH_3$, hydroxyalkyl, halogen, $(CH_2)_kNR''R'''$ where k = 0—2 and R″ and R‴ are independently hydrogen or $C_{1-6}$ alkyl; or, when attached to the 4- and 5-positions of the imidazole ring may be taken together to form a i) 5-, 6-, or 7-membered ring which may also contain a nitrogen atom; ii) benzene ring which is optionally substituted by halogen, hydroxy, $C_{1-6}$ alkyl, and $C_{1-6}$ alkoxy, and iii) pyridine ring; X is $(CH_2)_n$ or $O(CH_2)_{n+1}$ where n = 1—4; and the pharmaceutically acceptable salts thereof;

with the proviso that, when X is $(CH_2)_n$, when Y is a hydrogen, when A is attached to the 4-position of the phenyl ring, and when ---- in the general structural formula represents a single bond, then at least one of R, R′, $R_1$, $R_2$ and $R_3$ is other than a hydrogen atom:

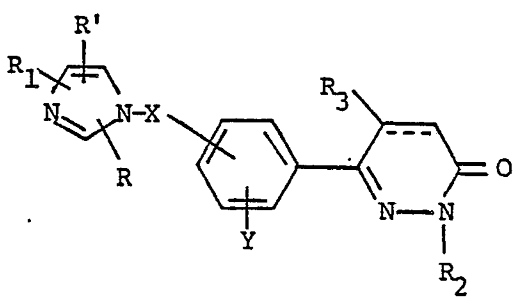

b. A =

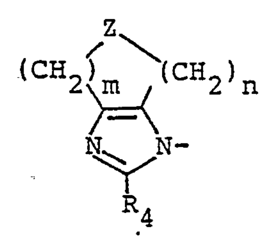

as in the structural formula (III) wherein m + n = 3, $R_4$ is hydrogen or $C_{1-6}$ alkyl; Z is $CH_2$ or $NR_5$ where $R_5$ is hydrogen, $C_{1-6}$ alkyl or $COR_6$ where $R_6$ is $C_{1-6}$ alkyl or aryl.

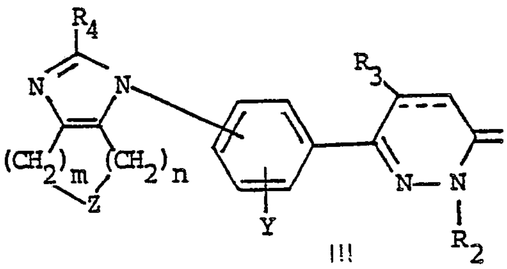

III

c. A =

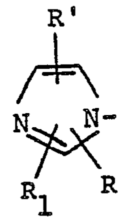

which is attached to 3-position of the phenyl ring as in the structural formula IV and R′, $R_1$, R, are same as defined in (a).

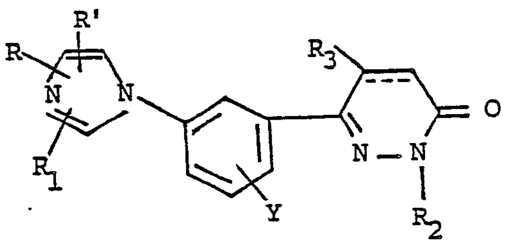

IV

d. A =

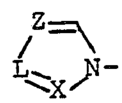

where
i) X = L = Z = CH as in the structural formula V
ii) X = Z = N and L = CH as in the structural formula VI
iii) L = Z = N and X = CH as in the structural formula VII

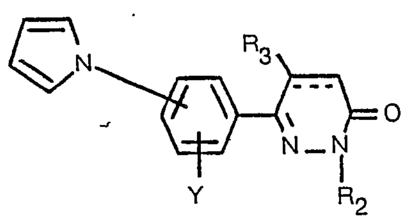

V

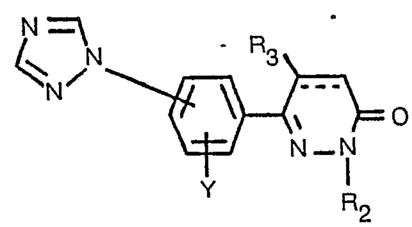

VI

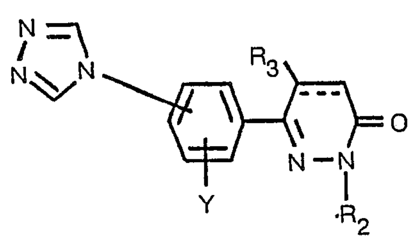

VII

e. Also included in the invention are novel pyridazinones having structural formula VIII where A is same as defined in (c) and is attached to the 4-position of the phenyl ring.

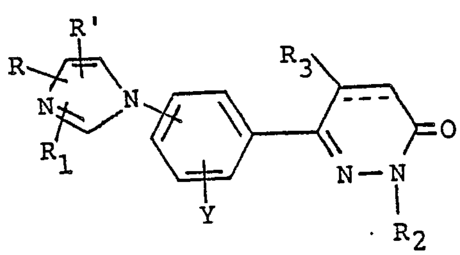

VIII

Examples of compounds of the invention are:

4,5-dihydro-6-[4-[2-1*H*-imidazol-1-yl)ethoxy]phenyl]-3(2*H*)-pyridazinone,
dihydro-6-[4-(4,5,6,7-tetrahydro-1*H*-benzimidazol-1-yl)-phenyl]-3(2*H*)-pyridazinone,
4,5-dihydro-6-[3-(1*H*-imidazol-1-yl)phenyl]-3(2*H*)-pyridazinone,
4,5-dihydro-6-[4-(1*H*-1,2,4-triazol-1-yl)phenyl]-3(2*H*)-pyridazinone,
4,5-dihydro-6-[4-(4*H*-1,2,4-triazol-4-yl)phenyl]-3(2*H*)-pyridazinone,
4,5-dihydro-6-[4-(1*H*-pyrrol-1-yl)phenyl]-3(2*H*)-pyridazinone,
4,5-dihydro-6-[4-(1*H*-imidazol-1-yl)phenyl)-3(2*H*)-pyridazinone,
4,5-dihydro-6-[4-(2-methyl-1*H*-imidazol-1-yl)-phenyl]-3(2*H*)-pyridazinone,
4,5-dihydro-6-[4-(2-ethyl-4-methyl-1*H*-imidazol-1-yl)phenyl)-3(2*H*)-pyridazinone,
4,5-dihydro-6-[4-(1*H*-imidazol-1-yl)phenyl]-5-methyl-3(2*H*)-pyridazinone,
4,5-dihydro-6-[4-(4,5-diethyl-1*H*-imidazol-1-yl)phenyl]-3(2*H*)-pyridazinone,
4,5-dihydro-6-[4-(1*H*-imidazol-1-yl)phenyl]-2-methyl-3(2*H*)-pyridazinone,
4,5-dihydro-6-[4-(1*H*-benzimidazol-1-yl)phenyl]-3(2*H*)-pyridazinone,
4,5-dihydro-6-[4-(4-hydroxymethyl-1*H*-imidazol-1-yl)phenyl-3(2*H*)-pyridazinone,
4,5-dihydro-6-[4-(2-methylthio-1*H*-imidazol-1-yl)phenyl]-3(2*H*)-pyridazinone,
4,5-dihydro-6-[4-(2-methylsulfinyl-1*H*-imidazol-1-yl)phenyl-3(2*H*)-pyridazinone,
4,5-dihydro-6-[4-(2-methylsulfonyl-1*H*-imidazol-1-yl)phenyl]-3(2*H*)-pyridazinone,
6-[4-(2-1*H*-imidazol-1-yl)ethoxy]phenyl-3(2*H*)-pyridazinone,
6-[4-[2-(1*H*-imidazol-1-yl)ethyl]phenyl]-3(2*H*)-pyridazinone,
6-[4-(1*H*-imidazol-1-yl-methyl)phenyl]-3(2*H*)-pyridazinone,
6-[4-(4,5,6,7-tetrahydro-1*H*-benzimidazol-1-yl)phenyl]-3(2*H*)-pyridazinone,
6-[3-(1*H*-imidazol-1-yl)phenyl]-3(2*H*)-pyridazinone,
6-[4-(1*H*-1,2,4-triazol-1-yl)phenyl]-3(2*H*)-pyridazinone,
6-[4-(4*H*-triazol-4-yl)phenyl]-3(2*H*)-pyridazinone,
6-[4-(1*H*-pyrrol-1-yl)phenyl]-3(2*H*)-pyridazinone,
6-[4-(1*H*-imidazol-1-yl)phenyl]-3(2*H*)-pyridazinone,
6-[4-(2-methyl-1*H*-imidazol-1-yl)phenyl-3(2*H*)-pyridazinone,
6-[4-(2-ethyl-4-methyl-1*H*-imidazol-1-yl)phenyl]-3(2*H*)-pyridazinone,
6-[4-(1*H*-imidazol-1-yl)phenyl-5-methyl-3(2*H*)-pyridazinone,
6-[4-(4,5-diethyl-1*H*-imidazol-1-yl)phenyl-3(2*H*)-pyridazinone,
6-[4-(4-hydroxymethyl-1*H*-imidazol-1-yl)phenyl-3(2*H*)-pyridazinone,
6-[4-(2-methylthio-1*H*-imidazol-1-yl)phenyl]-3(2*H*)-pyridazinone,
6-[4-(2-methylsulfinyl-1*H*-imidazol-1-yl)phenyl]-3(2*H*)-pyridazinone,
6-[4-(2-methylsulfonyl-1*H*-imidazol-1-yl)phenyl-3(2*H*)-pyridazinone,
6-[4-(1*H*-imidazol-1-yl)phenyl]-2-methyl-3(2*H*)-pyridazinone, and
6-[4-(1*H*-benzimidazol-1-yl)phenyl]-3(2*H*)-pyridazinone.

The invention also provides a process of producing the compounds according to the invention, having the structural formula:

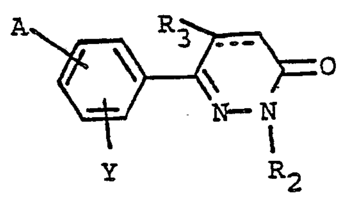

which comprises reacting a substituted γ-oxobenzenebutanoic acid having the structural formula:

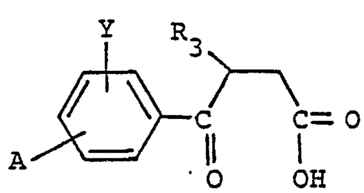

with a substituted hydrazine having the structural formula:

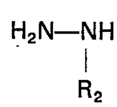

to obtain the compound having the structural formula

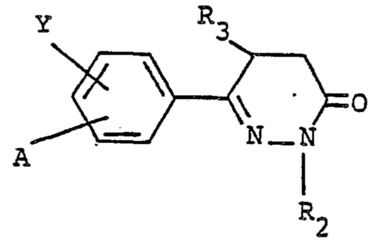

and when desired dehydrogenating to obtain the product

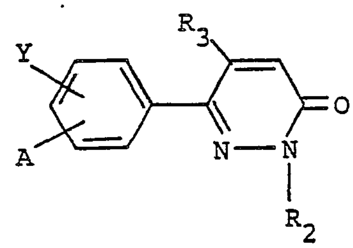

wherein A, Y, $R_2$, and $R_3$ are as defined above.

The invention also provides a pharmaceutical composition comprising a compound of the invention, and a pharmaceutically acceptable carrier or diluent therefor.

The compounds of formula I—VIII where $R_2$ is hydrogen may exist in tautomeric forms, for example, 6-[4-(1*H*-imidazol-1-yl)phenyl]-3(2*H*)-pyridazinones of formula VIII and/or 6-[4-(1*H*-imidazol-1-yl)phenyl]-3-pyridazinols of formula VIIIA, illustrated as follows.

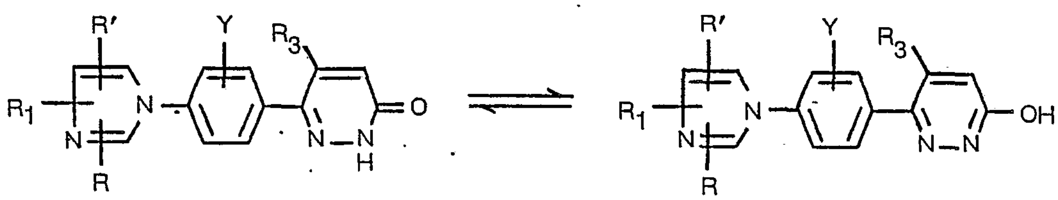

VIII VIII A

The present invention also relates to 4,5-dihydro-6-(substituted)phenyl-(3*H*)-pyridazinones having the structural formula IX:

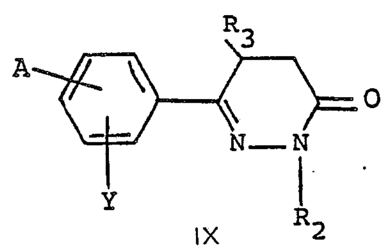

IX

wherein A, Y, $R_2$, and $R_3$ are same as defined above in the structural formulas I—VIII.

These compounds are not only useful as intermediates for preparing the compounds of formula I—VIII but are also useful as cardiotonic agents.

The compounds of the present invention may be used in a method for increasing cardiac contractility in a patient requiring such treatment, which comprises administering orally or parenterally in a solid or liquid dosage form to such patient an effective amount of compounds having the structures (I—VIII).

Another aspect of the present invention relates to a cardiotonic composition for increasing cardiac contractility, said composition comprising an effective amount of the compound of formula I—VIII and a pharmaceutically acceptable carrier.

The compounds of the present invention may be used in a method for increasing cardiac contractility, which comprises the administration of a medicament comprising an effective amount of the compound of formula I—VIII and a pharmaceutically acceptable carrier.

The process for producing pyridazinones (I—VIII) comprises reacting suitably substituted γ-oxobenzenebutanoic acids with suitably substituted hydrazines to give 4,5-dihydro-6-(substituted)phenyl-3(2*H*)-pyridazinones which can be dehydrogenated to the desired product by known dehydrogenation

procedures such as bromination-dehydrobromination; by noble metal catalyzed dehydrogenation such as palladium-catalyzed dehydrogenation or by oxidation-reduction procedures using $MnO_2$ or *m*-nitrobenzenesulphonic acid as the reagent according to the standard literature procedure set forth by W. V. Curran and A. Ross, *J. Med. Chem.*, *17*, 273 (1974).

The compounds of formula (I—VIII) are useful both in the free base form and in the form of acid addition salts. Both forms are within the scope of the invention. The acid addition salts are a more convenient form for use; and in practice, use of the salt form amounts to use of the base form. In practicing the invention, it was found convenient to form the sulfate, phosphate, or methanesulfonate salts. However, other appropriate pharmaceutically acceptable salts within the scope of the invention are those derived from mineral acids such as hydrochloric acid and sulfamic acid; and organic acids such as ethanesulfonic acid, benzenesulfonic acid, p-toluenesulfonic acid and the like, giving the hydrochloride, sulfamate, ethanesulfonate, benzenesulfonate, p-toluenesulfonate, and the like respectively.

The acid addition salts of said basic compounds are prepared either by dissolving the free base in aqueous or aqueous alcohol solution or other suitable solvents containing the appropriate acid and isolating the salt by evaporating the solution, or by reacting the free base and acid in an organic solvent, in which case the salt separates directly or can be obtained by concentration of the solution.

The following Examples 3 and 6 to 16 will further illustrate the invention without, however, limiting it thereto. The following Examples 1, 2, 3 and 4 relate to the preparation of intermediate compounds.

## Example 1

Methyl 4-(1*H*-Imidazol-1-yl)-β-oxobenzenepropanoate

A solution of 4-(1*H*-imidazol-1-yl)-acetophenone (24.2 g, 0.13 mol) in tetrahydrofuran (250 ml) is added to a suspension of 50% NaH (6.7 g) in tetrahydrofuran (70 ml) with stirring. The solution is stirred at room temperature for one hour. Dimethylcarbonate (30 ml) is added followed by refluxing the mixture overnight. The solid is filtered off, the residue is treated with water, and neutralized with acetic acid. The solid thus obtained is filtered off and crystallized from a methanol-ether mixture to yield 15.0 g of the product methyl-4-(1*H*-imidazol-1-yl)-β-oxobenzenepropanoate.

## Example 2

4-(1*H*-Imidazol-1-yl)-γ-oxobenzenebutanoic acid

A solution of methyl 4-(1*H*-imidazol-1-yl)-β-oxobenzenepropanoate (6.1 g, 0.025 mol) in tetrahydrofuran (65 ml) is added slowly to a stirred suspension of 50% NaH (1.2 g, 0.025 mol) in tetrahydrofuran (20 ml) and the solution is stirred for one additional hour. Ethyl bromoacetate (4.5 g) is added followed by refluxing the mixture for seven to eight hours. The tetrahydrofuran is removed, the residue is treated with water, and the organic material is extracted with ether. The residue obtained after removal of ether is hydrolysed by heating with 6N HCl for eight hours. The crude acid is finally crystallized from dimethylformamide to yield 3.3 g of the product 4-(1*H*-imidazol-1-yl)-γ-oxobenzenebutanoic acid.

## Example 3

4,5-Dihydro-6-[4-(1*H*-imidazol-1-yl)phenyl]-3(2*H*)-pyridazinone (3a)

A solution of 4.5 g of 4-(1*H*-imidazol-1-yl)-γ-oxobenzenebutanoic acid in ethanol (60 ml) is heated under reflux with 85% hydrazine hydrate (2.5 ml) for 17 hours. The alcohol is evaporated off, the residue is treated with water and filtered. The crude product is finally crystallized from ethanol to yield 3.5 g of the product 4,5-dihydro-6-[4-(1*H*-imidazol-1-yl)phenyl]-3(2*H*)-pyridazinone: m p 206—207°C (dec.).

| | | | |
|---|---|---|---|
| Anal calcd for $C_{13}H_{12}N_4O$: | C, 65.00; | H, 5.00; | N, 23.33 |
| Found: | C, 65.06; | H, 5.35; | N, 23.39 |

Similarly, the reaction of 4-(2-methyl-1*H*-imidazol-1-yl)-γ-oxobenzenebutanoic acid with hydrazine hydrate according to the procedure of this Example gives 4,5-dihydro-6-[4-(2-methyl-1*H*-imidazol-1-yl)phenyl]-3(2*H*)-pyridazinone (3b).

Similarly, the reaction of 4-(2-phenyl-1*H*-imidazol-1-yl)-γ-oxobenzenebutanoic acid with hydrazine hydrate according to the procedure of this Example gives 4,5-dihydro-6-[4-(2-phenyl-1*H*-imidazol-1-yl)-phenyl]-3(2*H*)-pyridazinone (3c).

Similarly, reaction of 4-(2-ethyl-4-methyl-1*H*-imidazol-1-yl)-γ-oxobenzenebutanoic acid with hydrazine hydrate according to the procedure of this Example gives 4,5-dihydro-6-[4-(2-ethyl-4-methyl-1*H*-imidazol-1-yl)phenyl]-3(2*H*)-pyridazinone (3d).

Using the procedure of this Example, reaction of 4-(1*H*-imidazol-1-yl)-γ-oxobenzenebutanoic acid with methyl hydrazine and 2-hydroxyethyl hydrazine gives 4,5-dihydro-6-[4-(1*H*-imidazol-1-yl)phenyl]-2-methyl-3(2*H*)-pyridazinone (3e) and 4,5-dihydro-2-(2-hydroxyethyl)-6-[4-(1*H*-imidazol-1-yl)phenyl]-3(2*H*)-pyridazinone (3f) respectively.

Similarly, the reaction of 4-(4-hydroxymethyl-1*H*-imidazol-1-yl)-γ-oxobenzenebutanoic acid with hydrazine hydrate according to the procedure of this Example gives 4,5-dihydro-6-[4-(4-hydroxymethyl-1*H*-imidazol-1-yl)phenyl]-3(2*H*)-pyridazinone (3g), mp 213.5—215°C.

Similarly, the reaction of 4-(4,5-diethyl-1*H*-imidazol-1-yl)-γ-oxobenzenebutanoic acid with hydrazine hydrate according to the procedure of this Example gives 4,5-dihydro-6-[4-(4,5-diethyl-1*H*-imidazol-1-yl)phenyl]-3(2*H*)-pyridazinone (3h).

Similarly, the reaction of 4-(1*H*-benzimidazol-1-yl)-γ-oxobenzenebutanoic acid with hydrazine hydrate according to the procedure of this Example gives 4,5-dihydro-6-[4-(1*H*-benzimidazol-1-yl)phenyl]-3(2*H*)-pyridazinone (3i), mp. 262—264°C.

4,5-Dihydro-6-[4-(1*H*-imidazol-1-yl)phenyl]-5-methyl-3(2*H*)-pyridazinone (3j)

A suspension of KCN (6.6 g in 8 ml of water) is added slowly to a stirred solution of a mixture of 4-(1*H*-imidazol-1-yl)benzaldehyde (17.2 g), p-toluenesulfonic acid (19 g) and morpholine (11.4 g) in dioxane (100 ml). The mixture is refluxed for three hours, concentrated to half its volume and poured into saturated $K_2CO_3$ solution. The oil is extracted with $CH_2Cl_2$, the $CH_2Cl_2$ extract is washed with water, dried, and evaporated to yield an oil which is filtered through silica gel. The oil is finally crystallized from ether.

To a stirred solution of the above [4-(1*H*-imidazol-1-yl)phenyl]-4-morpholineacetonitrile in THF (120 ml) is added 30 drops of 30% KOH in methanol followed by a slow addition of crotononitrile (4.2 g) over a period of 15 minutes and the resulting reaction mixture is stirred at room temperature for 90 minutes. The reaction mixture is concentrated in vacuo, the residue is treated with water and the oil is extracted with $CH_2Cl_2$. The methylene chloride extract is washed with water, dried, and concentrated to yield a highly viscous gum.

This is dissolved in 30 ml of 6N HCl and heated on a steam bath for six hours. The reddish solution is evaporated to dryness in vacuo, the residue is taken up in 150 ml of absolute ethanol, heated on a steam bath for 15 minutes and cooled. The inorganic salts are filtered off and the filtrate is directly used in the next step. The filtrate is heated to reflux with 85% hydrazine hydrate (4 ml) for four hours. The solution is cooled, diluted with water and filtered. The solid is crystallized from ethanol tetrahydrofuran to yield 2.8 g of the product 4,5-dihydro-6-[4-(1*H*-imidazol-1-yl)phenyl]-5-methyl-3(2*H*)-pyridazinone: (3j), mp 197—198°C.

| | | | |
|---|---|---|---|
| Anal calcd for $C_{14}H_{14}N_4O$: | C, 66.12; | H, 5.55; | N, 22.04 |
| Found: | C, 66.12; | H, 5.54; | N, 21.05. |

4,5-Dihydro-6-[4-(2-methylthio-1*H*-imidazol-1-yl)-phenyl]-3(2*H*)-pyridazinone (3k)

A solution of 6-(4-aminophenyl)-4,5-dihydro-3(2*H*)-pyridazinone (10 g, 0.053 mol) in N,N-dimethylformamide (200 ml) is added to an ice-cold solution of 1,1'-thiocarbonyldiimidazole (10 g, 0.056 mol) in N,N-dimethylformamide (50 ml) over a three hour period. The reaction mixture is slowly warmed up to room temperature and stirred for an additional half hour. The solution is diluted with 800 ml of water, cooled, filtered, and air-dried to give 10.8 g of the pure isothiocyanate, mp 181—182.5°C.

A solution of 10 g of the above isothiocyanate in N,N-dimethylformamide (60 ml) is added dropwise to a solution of aminoacetaldehyde diethyl acetal (7.17 g) in N,N-dimethylformamide (20 ml) followed by heating for two hours at 80°C. The DMF is removed by distillation under reduced pressure and the residue is heated to reflux with 100 ml of 10% HCl for one half hour. Upon cooling the solid is collected by filtration, washed with water, and finally crystallized to give 7 g of 4,5-dihydro-6-[4-(2-mercapto-1*H*-imidazol-1-yl)phenyl]-3(2*H*)-pyridazinone, mp 310—312.5° (dec).

A solution of the above compound (2.97 g) in N,N-dimethylformamide (50 ml) is treated with $CH_3I$ (4.6 g). The DMF is removed by distillation, and the residue is treated with water. The solution is then made basic and the crystalline material is collected by filtration to yield 1.75 g of the product 4,5-dihydro-6-[4-(2-methylthio-1*H*-imidazol-1-yl)phenyl]-3(2*H*)-pyridazinone, (3k), mp 155—156°C..

A solution of 4,5-dihydro-6-[4-(2-methylthio-1*H*-imidazol-1-yl)phenyl]-3(2*H*)-pyridazinone (2.08 g) in chloroform (30 ml) is oxidized with m-chloro perbenzoic acid (1.56 g) at 0°C to yield 1.45 g of the product 4,5-dihydro-6-[4-(2-methylsulfinyl-1*H*-imidazol-1-yl)-phenyl]-3(2*H*)-pyridazinone (3l), mp 187—188°C.

Similarly a solution of 4,5-dihydro-6-[4-(2-methylthio-1*H*-imidazol-1-yl)phenyl]-3(2*H*)-pyridazinone (1.68 g) in chloroform (15 ml) is oxidized with m-chloroperbenzoic acid (2.556 g) at ambient temperature to yield 1.54 g of the product 4,5-dihydro-6-[4-(2-methylsulfonyl-1*H*-imidazol-1-yl)phenyl]-3(2*H*)-pyridazinone, (3m), mp 200—201°C.

Example 4

Methyl-3-(1*H*-imidazol-1-yl)-β-oxobenzenepropanoate

A solution of 3-(1*H*-Imidazol-1-yl)acetophenone (53.4 g, 0.28 mol) in tetrahydrofuran (300 ml) is added to a suspension of 60% NaH (11.6 g) in tetrahydrofuran (90 ml) with stirring. The solution is stirred at room temperature for one hour. Dimethylcarbonate (70 ml) is added followed by refluxing the mixture overnight. The solid is filtered off, the residue is treated with water, neutralized with acetic acid and the oil is extracted with ethyl acetate. The ethyl acetate extract is filtered through silica gel and evaporated to yield 29.8 g of the product methyl 3-(1*H*-imidazol-1-yl)-β-oxobenzenepropanoate.

Example 5

3-(1*H*-Imidazol-1-yl)-γ-oxobenzenebutanoic acid

A solution of methyl-3-(1*H*-imidazol-1-yl)-β-oxobenzenepropanoate (29.1 g, 0.12 mol) in tetrahydrofuran (200 ml) is added slowly to a stirred suspension of 60% NaH (5.1 g, 0.12 mol) in

tetrahydrofuran (75 ml) and the solution is stirred for one additional hour. Ethyl bromoacetate (21.1 g) is added following by refluxing the mixture for seven to eight hours. The tetrahydrofuran is removed, the residue is treated with water, and the organic material is extracted with ether. The residue obtained after removal of ether is hydrolysed by heating with 6N HCl for eight hours. The crude acid is finally crystallized from water to yield 12.3 g of the product 3-(1*H*-imidazol-1-yl)-γ-oxobenzenebutanoic acid, mp 140.5—142°C.

Example 6

4,5-Dihydro-6-[3-(1-imidazol-1-yl)phenyl]-3(2*H*)-pyridazinone

A solution of 5.2 g of 3-(1*H*-imidazol-1-yl)-γ-oxobenzenebutanoic acid in ethanol (60 ml) is heated under reflux with 85% hydrazine hydrate (1.8 g) for four hours. The alcohol is evaporated off, the residue is treated with water and filtered. The crude product is finally crystallized from ethanol to yield 2.9 g of the product 4,5-dihydro-6-[3-(1*H*-imidazol-1-yl)phenyl]-3(2*H*)-pyridazinone, mp 190—190.5°C.

| | | | |
|---|---|---|---|
| Anal. Calcd for $C_{13}H_{12}N_4O$: | C, 64.98; | H, 5.03; | N, 23.39 |
| Found: | C, 65.02; | H, 5.08; | N, 23.33 |

Example 7

4-(4,5,6,7-Tetrahydro-1*H*-benzimidazol-1-yl)-γ-oxobenzenebutanoic acid

A solution of 4-fluoro-γ-oxobenzenebutanoic acid (20 g, 0.1 mol) and 5,6,7,8-tetrahydrobenzimidazole (12.2 g, 0.1 mol) in DMSO (50 ml) is added dropwise to a suspension of 50% NaH (9.6 g, 0.2 mol) in toluene (20 ml) with stirring keeping the temperature around 30°C. At this point additional DMSO (50 ml) is added and the mixture is stirred at room temperature overnight followed by heating at 100—110°C for 18 hours. The solution is cooled, extracted with ether and the aqueous solution is adjusted to pH ~5. The solid thus obtained is filtered, washed with water and crystallized from DMF to give 10 g of the product 4-(4,5,6,7-tetrahydro-1*H*-benzimidazol-1-yl)-γ-oxobenzenebutanoic acid, mp 234—235°C.

Example 8

4,5-Dihydro-6-[4-(4,5,6,7-tetrahydro-1*H*-benzimidazol-1-yl)phenyl]-3(2*H*)-pyridazinone

A mixture of 9 g of 4-(4,5,6,7-tetrahydro-1*H*-benzimidazol-1-yl)-γ-oxobenzenebutanoic acid and 3.5 g of hydrazine hydrate in 80 ml of ethanol is heated under reflux for six hours. The reaction mixture is allowed to cool and filtered. The crude product is finally crystallized from 2-methoxyethanol to yield 4.5 g of the product 4,5-dihydro-6-[4-(4,5,6,7-tetrahydro-1*H*-benzimidazol-1-yl)phenyl]-3(2*H*)-pyridazinone, mp 296—297°C.

| | | | |
|---|---|---|---|
| Anal Calcd. for $C_{17}H_{18}N_4O$, $0.1H_2O$: | C, 68.88; | H, 6.14; | N, 18.90 |
| Found: | C, 68.84; | H, 6.40; | N, 18.50 |

Example 9

4,5-Dihydro-6-[4-(1*H*-triazol-1-yl)phenyl]-3(2*H*)-pyridazinone

A solution of 15 g of 4-(1*H*-1,2,4-triazol-1-yl)-γ-oxobenzenebutanoic acid [mp 234—235°C, obtained from 4-(1*H*-1,2,4-triazol-1-yl)acetophenone according to the procedure of Examples 1 and 2] in ethanol (100 ml) is heated under reflux with 85% hydrazine hydrate (5.4 g) for six hours. The reaction mixture is allowed to cool and the solid is filtered, washed successively with dilute $NaHCO_3$ solution, water, and finally, crystallized from DMF to give 8.3 g of the product 4,5-Dihydro-6-[4-(1*H*-1,2,4-triazol-1-yl)phenyl]-3(2*H*)-pyridazinone, mp 311—312°C (dec.).

| | | | |
|---|---|---|---|
| Anal calcd for $C_{12}H_{11}N_5O$, 0.1 DMF: | C, 59.43; | H, 4.74; | N, 28.74 |
| Found: | C, 59.30; | H, 4.59; | N, 28.85 |

Example 10

4,5-Dihydro-(6-[4-(4*H*-1,2,4-triazol-4-yl)phenyl]-3(2*H*)-pyridazinone

A mixture of 6-(4-aminophenyl)-3(2*H*)-pyridazinone (3.78 g, 0.02 mol) and diformylhydrazine (1.76, 0.02 mol) are heated together at 220°C for six hours. The solid mass is chromatographed and finally crystallized from acetonitrile/methanol to yield the product 4,5-dihydro-6-[4-(4*H*-1,2,4-triazol-4-yl)phenyl]-3(2*H*)-pyridazinone, mp 292.5—293°C (dec).

| | | | |
|---|---|---|---|
| Anal calcd for $C_{12}H_{11}N_5O$: | C, 59.74; | H, 4.60; | N, 29.03 |
| Found: | C, 59.66; | H, 4.61; | N, 29.27 |

Example 11

4,5-Dihydro-6-[4-(1*H*-pyrrol-1-yl)phenyl]-3(2*H*)-pyridazinone

A mixture of 6-(4-aminophenyl)-3(2*H*)-pyridazinone (3.7 g) and 2,5-dimethoxytetrahydrofuran (2.6 g) in glacial acetic acid (37 ml) is heated under reflux for four hours. The reaction mixture is cooled, filtered and

the solid is washed with ethanol, and finally crystallized from methanol to give 1.2 g of the product. 4,5-Dihydro-6-[4-(1*H*)-pyrrol-1-yl)phenyl]-3(2*H*)-pyridazinone, mp 222—223°C.

| | | | |
|---|---|---|---|
| Anal calcd for $C_{14}H_{13}N_3O$: | C, 70.27; | H, 5.48; | N, 17.56 |
| Found: | C, 70.50; | H, 5.40; | N, 17.60 |

Example 12

4,5-Dihydro-6-[4-[2-(1*H*-imidazol-1-yl)ethoxy]phenyl]-3(2*H*)-pyridazinone

Potassium-t-butoxide (0.56 g, 0.5 mmol) is added to a solution of imidazole (0.34 g, 0.5 mmol) in dry DMF (10 ml) under $N_2$. The resulting mixture is stirred until homogenous and then five minutes longer. A solution of 4,5-dihydro-6-(2-chloroethoxyphenyl)-3(2*H*)-pyridazinone (1.27 g, 0.5 mmol) in dry DMF (20 ml) is added in one portion and the resultant mixture is heated and stirred at 60° for 12 hours. The DMF is then distilled off and the residue taken up in chloroform. The chloroform solution is extracted with 5% aqueous HCl. The aqueous extract is made basic with 10% aq sodium carbonate and extracted several times with methylene chloride. The methylene chloride is evaporated, leaving behind 0.45 g of the product 4,5-dihydro-6-[4-[2-(1*H*-imidazol-1-yl)ethoxyphenyl]-3(2*H*)-pyridazinone as an amorphous solid.

Example 13

6-[4-(1*H*-Imidazol-1-yl)phenyl]-3(2*H*)-pyridazinone (13a)

Bromine (1.6 ml) is added dropwise to a solution of 4,5-dihydro-6-[4-(1*H*-imidazol-1-yl)phenyl]-3(2*H*)-pyridazinone (3.5 g) in acetic acid (25 ml) at 80°C. The mixture is heated for six hours to complete the reaction. The solid is filtered, washed with ether and converted to the free base which is crystallized from ethanol to yield 1.1 g of the product 6-[4-(1*H*-imidazol-1-yl)phenyl]-3(2*H*)-pyridazinone, mp 244—245°C.

| | | | | |
|---|---|---|---|---|
| Anal calcd for $C_{13}H_{10}N_4O,1/5H_2O$: | C, 64.56; | H, 4.30; | N, 23.17; | $H_2O$, 1.49 |
| Found: | C, 64.30; | H, 4.36; | N, 23.04; | $H_2O$, 1.11 |

Similarly, reaction of 4,5-dihydro-6-[4-(2-methyl-1*H*-imidazol-1-yl)phenyl]-3(2*H*)-pyridazinone with bromine in acetic acid as described in this Example gives 6-[4-(2-methyl-1*H*-imidazol-1-yl)phenyl-3(2*H*)-pyridazinone (13b).

Similarly, reaction of 4,5-dihydro-6-[4-(2-phenyl-1*H*-imidazol-1-yl)phenyl-3(2*H*)-pyridazinone with bromine in acetic acid as described in this Example gives 6-[4-(2-phenyl-1*H*-imidazol-1-yl)phenyl]-3(2*H*)-pyridazinone (13c).

Similarly, reaction of 4,5-dihydro-6-[4-(2-ethyl-4-methyl-1*H*-imidazol-1-yl)phenyl]-3(2*H*)-pyridazinone with bromine in acetic acid as described in this Example gives 6-[4-(2-ethyl-4-methyl-3(2*H*)-pyridazinone (13d).

Using the procedure of this Example, reaction of 4,5-dihydro-2-methyl-6-[4-(1*H*-imidazol-1-yl)phenyl]-3(2*H*)-pyridazinone and 4,5-dihydro-2-(2-hydroxyethyl)-6-[4-(1*H*-imidazol-1-yl)phenyl]-3(2*H*)-pyridazinone with bromine in acetic acid gives 6-[4-(1*H*-imidazol-1-yl)phenyl]-2-methyl-3(2*H*)-pyridazinone (13e) and 2-(2-hydroxyethyl)-6-[4-(1*H*-imidazol-1-yl)phenyl]-3(2*H*)-pyridazinone respectively (13f).

Similarly, reaction of 4,5-dihydro-6-[4-(4,5-diethyl-1*H*-imidazol-1-yl)phenyl]-3(2*H*)-pyridazinone with bromine in acetic acid as described in this Example gives 6-[4-(4,5-diethyl-1*H*-imidazol-1-yl)phenyl)-3(2*H*)-pyridazinone (13g).

Similarly, reaction of 4,5-dihydro-6-[4-(1*H*-benzimidazol-1-yl)phenyl]-3(2*H*)-pyridazinone with bromine in acetic acid as described in this Example gives 6-[4-1*H*-benzimidazol-1-yl)phenyl)-3(2*H*)-pyridazinone (13h).

6-[4-(1*H*-Imidazol-1-yl)phenyl]-5-methyl-3(2*H*)-pyridazinone (13i)

A solution of 3.6 g of 4,5-dihydro-6-[4-(1*H*-imidazol-1-yl)phenyl]-5-methyl-3(2*H*)-pyridazinone in a mixture of dioxane (100 ml) and N,N-dimethylformamide (25 ml) is heated with 12 g of $MnO_2$ at 90°C overnight. The temperature is raised to 105°C and maintained there for four hours. The inorganic solid is filtered off and washed thoroughly with hot dioxane. The filtrate and the washings are combined, evaporated in vacuo, and the residue is crystallized from methanol and tetrahydrofuran to yield 2.1 g of the product. 6-[4-(1*H*-imidazol-1-yl)phenyl]-5-methyl-3(2*H*)-pyridazinone, mp 284—286°C.

| | | | |
|---|---|---|---|
| Anal calcd for $C_{14}H_{12}N_4O$: | C, 66.65; | H, 4.79; | N, 22.21 |
| Found: | C, 66.22; | H, 4.59; | N, 22.06 |

Example 14

6-[3-(1*H*-Imidazol-1-yl)phenyl]-3(2*H*)-pyridazinone

A solution of bromine (0.7 ml) in acetic acid (20 ml) is added dropwise to a solution of 2.6 g of 4,5-dihydro-6-[3-(1*H*-imidazol-1-yl)phenyl-3(2*H*)-pyridazinone in acetic acid (85 ml) at 90—95°. The reaction mixture is heated to reflux for 3.5 hours. Upon cooling the solid is filtered, washed with ether and converted

to the free base which is crystallized from ethanol to yield 1.3 g of the product 6-[3-(1*H*-imidazol-1-yl)phenyl]-3(2*H*)-pyridazinone, mp 234.5—235.5°C.

| | | | |
|---|---|---|---|
| Anal calcd for $C_{13}H_{10}N_4O$: | C, 65.53; | H, 4.23; | N, 23.52 |
| Found: | C, 65.44; | H, 4.66; | N, 23.73 |

Example 15

6-[4-(4,5,6,7-Tetrahydro-1*H*-benzimidazol-1-yl)phenyl]-3(2*H*)-pyridazinone

A solution of bromine (1 g) in 10 ml of acetic acid is added dropwise to a solution of 1.6 g of 4,5-dihydro-6-[4-(4,5,6,7-tetrahydro-1*H*-benzimidazol-1-yl)phenyl]-3(2*H*)-pyridazinone in 40 ml of acetic acid at 86—88°C. The mixture is subsequently heated at 100°C for four to five hours. The reaction mixture is cooled, the solid is filtered off, washed with ether, and air dried. The hydrobromide salt thus obtained is converted to the free base which is crystallized from ethanol to give 0.6 g of the product 6-[4-(4,5,6,7-tetrahydro-1*H*-benzimidazol-1-yl)phenyl]-3(2*H*)-pyridazinone, mp 266—267°C.

| | | | |
|---|---|---|---|
| Anal calcd for $C_{17}H_{16}N_4O,0.1H_2O$: | C, 69.35; | H, 5.50; | N, 19.03 |
| Found: | C, 69.25; | H, 5.36; | N, 19.03 |

Example 16

6-[4-[2-(1*H*-Imidazol-1-yl)ethoxy]phenyl]-3(2*H*)-pyridazinone

A solution of bromine (0.6 g) in acetic acid (6 ml) is added dropwise to a solution of 4,5-dihydro-6-[4-[2-(1*H*-imidazol-1-yl)ethoxy)phenyl]-3(2*H*)-pyridazinone (2.84, 0.01 mol) in acetic acid (56 ml) at 90°C. The mixture is subsequently heated at 100°C for four hours. The reaction mixture is cooled, the solid is filtered off, washed with ether, and air dried. The hydrobromide salt thus obtained is converted to the free base which is crystallized from ethanol to give 1.2 g of the product 6-[4-[2-(1*H*-imidazol-1-yl)ethoxy]phenyl]-3(2*H*)-pyridazinone.

The usefulness of the compounds of the present invention as cardiotonic agents is demonstrated by their effectiveness in standard pharmacological test procedures, for example, in causing a significant increase in the myocardial contractility in the pentobarbital-anesthetized dog with low or minimal changes in heart rate and blood pressure. This test procedure is described in the following paragraphs.

Test for in vivo myocardial inotropic activity in anesthetized dog

This screen consists of determining the effects of increasing intravenous doses of compound on myocardial contractility (dP/dt mad of left ventricular blood pressure), heart rate and aortic blood pressure of the pentobarbital-anesthetized dog.

Methods

Adult mongrel dogs of either sex are anesthetized with pentobarbital, 35 mg/kg, IV, and are subsequently maintained under anesthesia with a continuous infusion of pentabarbital, 3.5 mg/kg/hour. The trachea is intubated but the animals are permitted to breathe spontaneously. A cannula is inserted into the femoral vein for administrating test agents. A Millar catheter tip pressure transducer or a fluid filled catheter is inserted into the ascending aorta via the femoral artery for measuring aortic blood pressure. A Millar catheter tip pressure transducer is passed into the left ventricle via the left carotid artery for measuring left ventricular blood pressure. Needle electrodes are placed subcutaneously for recording a lead II electrocardiogram (ECG).

Left ventricular and aortic blood pressures are recorded on a strip chart recorder. Heart rate, using a biotachometer triggered from the R wave of the ECG, and the first derivative of left ventricular blood pressure (dP/dt), obtained with a differentiator amplifier coupled to the corresponding pressure amplifier, are also recorded. A period of at least 30 minutes is utilized to obtain control data prior to administration of test compound.

Depending on solubility, the compounds are dissolved in 0.9% saline solution or in dilute HCl or NaOH (0.1 or 1.0 N) and are diluted to volume with normal saline. Ethanol or dimethylacetamide can be used as solvents if adequate dilutions can be made. Appropriate vehicle controls are administered when needed.

Each dose of the test compound is administered in volume of 0.1 ml/kg over a period of one minute.

When tested by the above-described Anesthetized Dog Procedure, the compounds of the present invention when administered intravenously at a rate of about 0.01 to 0.31 mg/kg/min cause dose related significant increases in cardiac contractility with only low or minimal changes in heart rate and blood pressure.

Test Results of 4,5-Dihydro-6-(1*H*-imidazol-1-yl)phenyl]-3(2*H*)-pyridazinone using anesthetized dog procedure

% Change

| Example | Dose mg/kg | Myocardial Contractility | Heart Rate | Blood Pressure |
|---|---|---|---|---|
| 3a | 0.01 | 9 | −4.0 | −2.0 |
| | 0.03 | 32 | −4.0 | −6.0 |
| | 0.10 | 57 | −1.0 | −10.5 |
| | 0.31 | 87 | 2.0 | −21.5 |
| 6 | 0.01 | 6 | −2.0 | 1.0 |
| | 0.10 | 36 | −1.0 | −1.0 |
| | 0.30 | 71 | 2.0 | −8.0 |
| | 1.0 | 114 | 11.0 | −20.0 |
| 8 | 0.01 | 10 | −3.0 | −2.5 |
| | 0.03 | 21 | −15.0 | −7.0 |
| | 0.10 | 51 | −17.0 | −11.0 |
| | 0.31 | 95 | −18.0 | −17.0 |
| | 1.0 | 127 | −7.0 | −27.0 |

Test results of 6-(1*H*-Imidazol-1-yl)phenyl]-3(2*H*)-pyridazinone using anesthetized dog procedure

% Change

| Example | Dose mg/kg | Myocardial Contractility | Heart Rate | Blood Pressure |
|---|---|---|---|---|
| 13a | 0.01 | 3 | 0.0 | 0.0 |
| | 0.03 | 15 | 6.0 | −1.5 |
| | 0.10 | 40 | 16.0 | −6.0 |
| | 0.31 | 78 | 32.0 | −9.0 |

The usefulness of the compounds of the present invention as antihypertensive agents is demonstrated by their effectiveness in standard pharmacological test procedures, for example, in causing a significant decrease in mean arterial blood pressure in conscious rat. This test procedure is described in the following paragraphs.

A method for the direct monitoring of aortic blood pressure and heart rate from conscious rats

The continuous monitoring of pulsatile blood pressure from unrestrained conscious rats surgically equipped with polyethylene cannulas was accomplished by means of a computer assisted data capture scheme (CADCS). The basic elements of the methodology are the cannulation procedure and the CADCS.

Method

*Cannulation Procedure:* Rats were anesthetized with Telazol (1:1 tiletamine HCl and zolazepam HCl; WL/PD, Ann Arbor, MI) 20—40 mg/kg IM and the descending aorta exposed via a midline incision. Cannulus fabricated from polyethylene tubing (Clay Adams, Parsippany, NJ) were inserted into the aorta via an undersized puncture hole below the renal arteries. The puncture hole was made by a 23 G disposable needle with a section of the aorta clamped off above and below the puncture site. The cannulas, consisting of a PE100 (0.86 mm ID) body and a PE50 (0.58 mm ID) tip, were attached to a trocar, inserted through the psoas muscle, and passed subcutaneously along the midline of the back and externalized between the ears. The cannulas were anchored to the psoas muscle and between the scapulae (3—0 green braided suture; Deknatel, Queens Village, NY). The midline incision was closed in two steps (muscle first, skin second) using continuous over-and-over sutures (4—0 chronic; Ethicon, Somerville, NJ). Each rat was then given penicillin 30,000 units subcutaneously (Penicillin G Procaine Sterile Suspension; Parke-Davis, Detroit, MI).

The rats were fitted with a harness-spring-swivel assembly designed to protect the cannula and to provide the rat relative freedom of movement. The harnesses were fabricated from nylon hook and loop tape (Velcro, Manchester, NH) cemented to a metal plate to which spring wires (18—8 stainless steel, Paragon Spring; Chicago, IL) were attached to brass swivels (BRS/LVE, Bellville, MD). Each polyethylene cannula was channeled through a spring and connected through a swivel to a pressure transducer (Model P23Gb; Statham Instruments; Hato Rey, Puerto Rico) and an infusion pump (Sage model 234—7; Orion Research, Cambridge, MA) by means of PE100 tubing. While on test, each rat received a continuous slow infusion of heparinized saline solution (approximately 400) l or 40 units of heparin per 24-hour period) to prevent clot formation. Additional "flushes" of the cannula with heparinized saline were carriedd out when the aortic pulse pressure (systolic minus diastolic) was less than 25 mm Hg.

CADCS: The pulsatile blood pressure and heart rate of each of 32 rats was monitored every minute by means of two in-laboratory microcomputers communicating directly with a data concentrator computer. The data were first stored on the data concentrator disk and then transferred to a magnetic tape for analysis and report generation by the main research computer (Varian V-74 or IBM). The overall scheme involved modulating the primary signal from the pressure transducer, generating the primary data set of the one-minute values for systolic, diastolic, and mean blood pressures and heart rate by the in-lab microcomputer and the storage, analysis, and report generation by the main research computer.

The transducers were connected to analog signal conditioning modules. The modules provided a regulated excitation voltage for the transducers, amplification as required to interface the microprocessors and an active low pass filter to compensate for the pressure wave form distortion produced by the flexible, fluid filled, narrow lumened cannula. The distortion was 22—26 Hz and thus provided a reliable estimate of both systolic and diastolic blood pressure.

The microcomputers (one for each of two groups of 16 rats) were connected to the input components through the module interface units, an analog-to-digital converter for the pressure wave form signal and the digital inputs for the dose and event marker switches. The microcomputer controlled the sequential acquisition of data from the modular interface units through an internal synchronous time-of-day clock/time base generator. Utilizing the time base generator as a reference, the blood pressure values and the marker switch status for each of the 32 stations were sampled every 10 msec. The microcomputer processed each blood pressure sample as it was received to produce "running average" values for heart rate, and mean, systolic and diastolic blood pressures.

When tested by the above procedure the compound of Formula I—VIII or pharmaceutically acceptable acid-addition salts thereof, e.g., Example 3 (a-m), 6, 8, 9, 10, 11, 12 at doses 1, 3, 10, and 30 mg/kg were found to cause a significant reduction in aortic blood pressure.

When tested by the above procedure the compound of Formula I—VIII or pharmaceutically acceptable acid-addition salts thereof, e.g., Example 13 (a-i), 14, 15, 16 at doses 1, 3, 10, and 30 mg/kg were found to cause a significant reduction in aortic blood pressure.

The actual determination of the numerical cardiotonic data definitive for any other particular compound of the invention is readily obtained according to the above-described standard test procedure by those skilled in pharmacological test procedures, without any need for any extensive experimentation.

The present invention includes within its scope a cardiotonic composition for increasing cardiac contractility, said composition comprising a pharmaceutically acceptable carrier and, as the active component thereof, a cardiotonic compound of the present invention or pharmaceutically acceptable acid addition salt thereof. The invention also includes within its scope the method for increasing cardiac contractility in a patient requiring such treatment which comprises administering to such patient an effective amount of a compound of the present invention or pharmaceutically acceptable acid addition salt thereof. In clinical practice the said compounds of the present invention will normally be administered orally or parenterally in a wide variety of dosage forms.

Solid compositions for oral administration include compressed tablets, pills, powders, and granules. In such solid compositions, at least one of the active compounds is admixed with at least one inert diluent such as starch, calcium carbonate, sucrose, or lactose. These compositions may also contain additional substances other than inert diluents, e.g., lubricating agents such as magnesium stearate, talc, and the like.

Liquid compositions for oral administration include pharmaceutically acceptable emulsions, solutions, suspensions, syrups, and elixirs containing inert diluents commonly used in the art, such as water or liquid

paraffin. Besides inert diluents such compositions may also contain adjuvants, such as wetting and suspending agents, and sweetening, flavoring, perfuming, and preserving agents. According to the invention, the compounds for oral administration also include capsules of absorbable material, such as gelatin, containing said active component with or without the addition of diluents or excipients.

Preparations according to the invention for parenteral administration include sterile aqueous, aqueous organic, and organic solutions, suspensions and emulsions. Examples of organic solvents or suspending media are propylene glycol, polyethylene glycol, vegetable oils such as olive oil and injectable organic esters such as ethyl oleate. These compositions may also contain adjuvants such as stabilizing, preserving, wetting, emulsifying, and dispersing agents.

They may be sterilized, for example by filtration through a bacteria-retaining filter, by incorporation of sterilizing agents in the compositions, by irradiation or by heating. They may also be manufactured in the form of sterile solid compositions which can be dissolved in sterile water or some sterile injectable medium immediately before use.

The percentage of active component in the said composition and method for increasing cardiac contractility may be varied so that a suitable dosage is obtained. The dosage administered to a particular patient is variable, depending upon the clinician's judgment using as the criteria: The route of administration, the duration of treatment, the size and condition of the patient, the potency of the active compound and the patient's response thereto. An effective dosage amount of active component can thus only be determined by the clinician considering all criteria and utilizing his best judgment on the patient's behalf.

**Claims for the Contracting States: BE CH DE FR GB IT LI LU NL SE**

1. A compound having the structural formula:

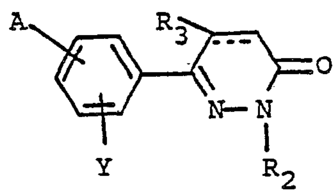

wherein ---- represents a double or single bond between two carbon atoms; $R_2$ and $R_3$ are independently hydrogen or $C_{1-6}$ alkyl; Y is H, halogen, $C_{1-6}$ alkyl, and $C_{1-6}$ alkoxy, and A is any of the groups from a—e, and is attached to the 3- or 4-position of the phenyl ring

a. A = 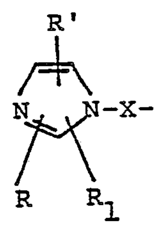

where X is $(CH_2)_n$ or $O(CH_2)_{n+1}$ where n = 1—4; $R_1$, R', and R are independently hydrogen or $C_{1-6}$ alkyl; hydroxyalkyl, halogen, $CH_2OH$, $SCH_3$, $SOCH_3$, $SO_2CH_3$, $(CH_2)_kNR''R'''$, where k = 0—2 and R'' and R''' are independently hydrogen or $C_{1-6}$ alkyl, or when attached to the 4- and 5-positions of the imidazole ring may be taken together to form a i) 5-, 6-, or 7-membered ring which may also contain a nitrogen atom; ii) benzene ring which is optionally substituted by halogen, hydroxy, $C_{1-6}$ alkyl, and $C_{1-6}$ alkoxy, and iii) pyridine ring; and the pharmaceutically acceptable salts thereof;
with the proviso that, when X is $(CH_2)_n$, when Y is a hydrogen, when A is attached to the 4-position of the phenyl ring, and when ---- in the general structural formula represents a single bond, then at least one of R, R', $R_1$, $R_2$ and $R_3$ is other than a hydrogen atom:

b. A = 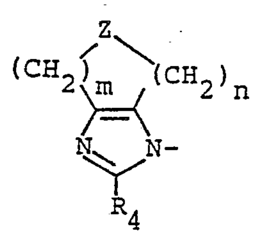

where m + n = 3, $R_4$ is hydrogen or $C_{1-6}$ alkyl; Z is $CH_2$ or $NR_5$ where $R_5$ is hydrogen, $C_{1-6}$ alkyl or $COR_6$ where $R_6$ is $C_{1-6}$ alkyl or aryl and the pharmaceutically acceptable salts thereof

c. A = 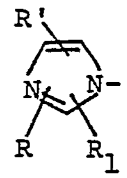

where $R_1$, R′, and R are as defined in (a) and is attached to the 3-position of the phenyl ring and the pharmaceutically acceptable salts thereof

d. A = 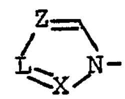

where i) X = L = Z = CH; ii) X = Z = N, and L=CH iii) L = Z = N and X = CH and their pharmaceutically acceptable salts thereof

e. A is same as in I(c) and is attached to the 4-position of the phenyl ring and the pharmaceutically acceptable salts thereof.

2. A compound according to Claim 1 having the structure:

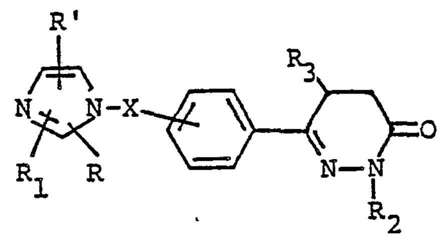

wherein $R_1$, R′, R, X, $R_2$, and $R_3$ are as defined in Claim 1a, and the pharmaceutically acceptable salts thereof.

3. A compound according to Claim 1 having the structure:

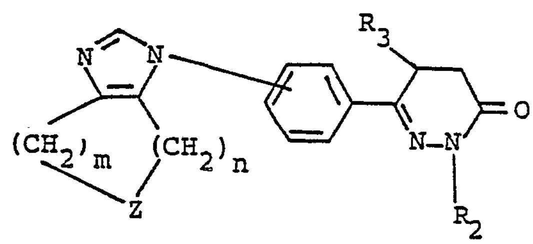

wherein $R_4$, $R_2$, $R_3$, Z, m, and n are as defined in Claim Ib, and the pharmaceutically acceptable salts thereof.

4. A compound according to Claim 1 having the structure:

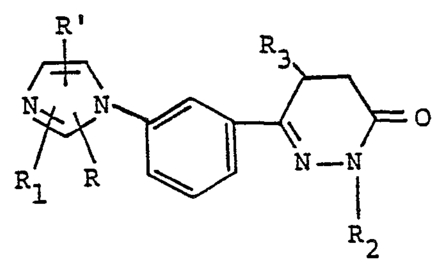

wherein $R_1$, R′, R, $R_2$, and $R_3$ are as defined in Claim 1c, and the pharmaceutically acceptable salts thereof.

5. A compound according to Claim 1 having the structure:

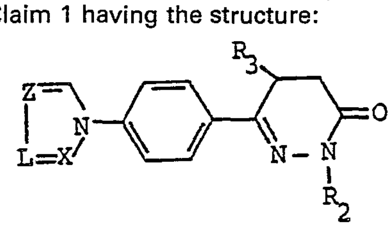

wherein X, L, Z, $R_2$, and $R_3$ are as defined in Claim 1d, and the pharmaceutically acceptable salts thereof.

6. A compound according to Claim 1 having the structure:

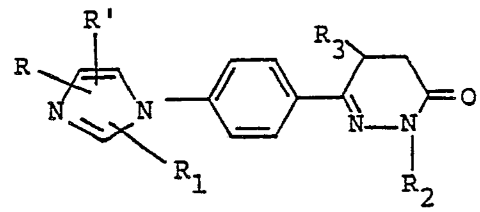

wherein $R_1$, R, R′, $R_2$, and $R_3$ are as defined in Claim 1e, and the pharmaceutically acceptable salts thereof.

7. A compound according to Claim 1 having the structure:

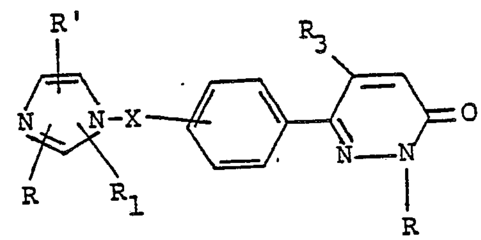

wherein $R_1$, R′, R, X, $R_2$, and $R_3$ are as defined in Claim 1a, and the pharmaceutically acceptable salts thereof.

8. A compound according to Claim 1 having the structure:

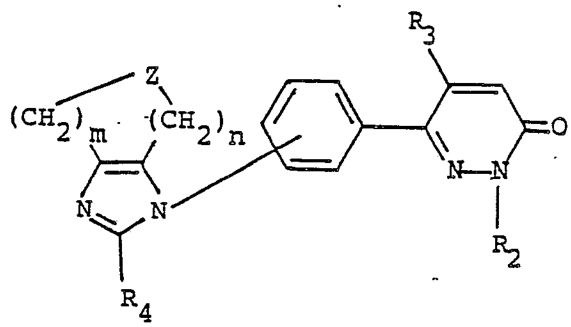

wherein $R_2$, $R_3$, $R_4$, Z, m, and n are as defined in Claim 1b, and the pharmaceutically acceptable salts thereof.

9. A compound according to Claim 1 having the structure:

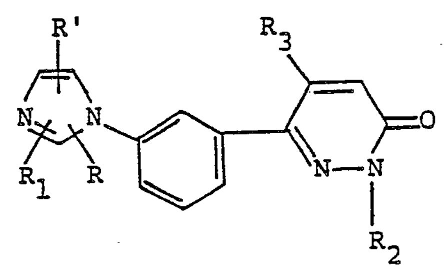

wherein $R_1$, R′, R, $R_2$, and $R_3$ are as defined in Claim 1c, and the pharmaceutically acceptable salts thereof.

10. A compound according to Claim 1, having the structure:

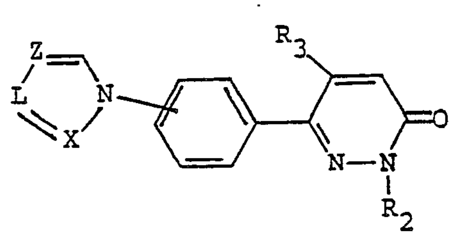

wherein X, L, Z, $R_2$, and $R_3$ are as defined in Claim 1d, and the pharmaceutically acceptable salts thereof.

11. A compound according to Claim 1, having the structure:

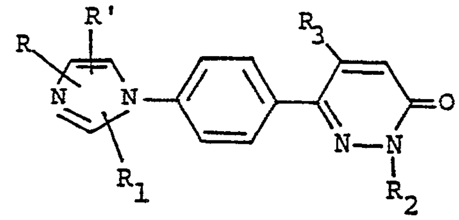

wherein $R_1$, R, R′, $R_2$, and $R_3$ are as defined in Claim 1e and the pharmaceutically acceptable salts thereof.

12. A compound according to Claim 1, which is
4,5-dihydro-6-[4-[2-1*H*-imidazol-1-yl)ethoxy]phenyl]-3(2*H*)-pyridazinone,
4,5-dihydro-6-[4-(4,5,6,7-tetrahydro-1*H*-benzimidazol-1-yl)-phenyl]-3(2*H*)-pyridazinone,
4,5-dihydro-6-[3-(1*H*-imidazol-1-yl)phenyl]-3(2*H*)-pyridazinone,
4,5-dihydro-6-[4-(1*H*-1,2,4-triazol-1-yl)phenyl]-3(2*H*)-pyridazinone,
4,5-dihydro-6-[4-(4*H*-1,2,4-triazol-4-yl)phenyl]-3(2*H*)-pyridazinone,
4,5-dihydro-6-[4-(1*H*-pyrrol-1-yl)phenyl]-3(2*H*)-pyridazinone,
4,5-dihydro-6-[4-(1*H*-imidazol-1-yl)phenyl]-3(2*H*)-pyridazinone,
4,5-dihydro-6-[4-(2-methyl-1*H*-imidazol-1-yl)phenyl]-3(2*H*)-pyridazinone,
4,5-dihydro-6-[4-(2-ethyl-4-methyl-1*H*-imidazol-1-yl)phenyl]-3(2*H*)-pyridazinone,
4,5-dihydro-6-[4-(1*H*-imidazol-1-yl)phenyl]-5-methyl-3(2*H*)-pyridazinone,
4,5-dihydro-6-[4-(4,5-diethyl-1*H*-imidazol-1-yl)phenyl]-3(2*H*)-pyridazinone,
4,5-dihydro-6-[4-(1*H*-imidazol-1-yl)phenyl]-2-methyl-3(2*H*)-pyridazinone,
4,5-dihydro-6-[4-(1*H*-benzimidazol-1-yl)phenyl]-3(2*H*)-pyridazinone,
4,5-dihydro-6-[4-(4-hydroxymethyl-1*H*-imidazol-1-yl)phenyl]-3(2*H*)-pyridazinone,
4,5-dihydro-6-[4-(2-methylthio-1*H*-imidazol-1-yl)phenyl]-3(2*H*)-pyridazinone,
4,5-dihydro-6-[4-(2-methylsulfinyl-1*H*-imidazol-1-yl)phenyl]-3(2*H*)-pyridazinone,
4,5-dihydro-6-[4-(2-methylsulfonyl-1*H*-imidazol-1-yl)phenyl]-3(2*H*)-pyridazinone,
6-[4-(2-1*H*-imidazol-1-yl)ethoxy]phenyl]-3(2*H*)-pyridazinone,
6-[4-[2-(1*H*-imidazol-1-yl)ethyl]phenyl]-3(2*H*)-pyridazinone,
6-[4-(1*H*-imidazol-1-yl-methyl)phenyl]-3(2*H*)-pyridazinone,
6-[4-(4,5,6,7-tetrahydro-1*H*-benzimidazol-1-yl)phenyl]-3(2*H*)-pyridazinone,
6-[3-(1*H*-imidazol-1-yl)phenyl]-3(2*H*)-pyridazinone,
6-[4-(1*H*-1,2,4-triazol-1-yl)phenyl]-3(2*H*)-pyridazinone,
6-[4-(4*H*-1,2,4-triazol-4-yl)phenyl]-3(2*H*)-pyridazinone,
6-[4-(1*H*-pyrrol-1-yl)phenyl]-3(2*H*)-pyridazinone,
6-[4-(1*H*-imidazol-1-yl)phenyl]-3(2*H*)-pyridazinone,
6-[4-(2-methyl-1*H*-imidazol-1-yl)phenyl]-3(2*H*)-pyridazinone,
6-[4-(2-ethyl-4-methyl-1*H*-imidazol-1-yl)phenyl]-3(2*H*)-pyridazinone,
6-[4-(1*H*-imidazol-1-yl)phenyl]-5-methyl-3(2*H*)-pyridazinone,
6-[4-(4,5-diethyl-1*H*-imidazol-1-yl)phenyl]-3(2*H*)-pyridazinone,
6-[4-(4-hydroxymethyl-1*H*-imidazol-1-yl)phenyl]-3(2*H*)-pyridazinone,
6-[4-(2-methylthio-1*H*-imidazol-1-yl)phenyl]-3(2*H*)-pyridazinone,
6-[4-(2-methylsulfinyl-1*H*-imidazol-1-yl)phenyl]-3(2*H*)-pyridazinone,
6-[4-(2-methylsulfonyl-1*H*-imidazol-1-yl)phenyl]-3(2*H*)-pyridazinone,
6-[4-(1*H*-imidazol-1-yl)phenyl]-2-methyl-3(2*H*)-pyridazinone, or
6-[4-(1*H*-benzimidazol-1-yl)phenyl]-3(2*H*)-pyridazinone.

13. A process of producing the compounds according to Claim 1 having the structural formula:

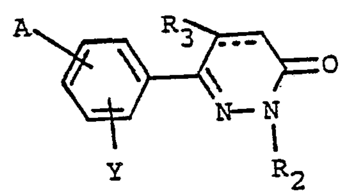

which comprises reacting a substituted γ-oxobenzenebutanoic acid having the structural formula:

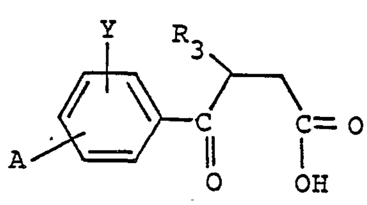

with a substituted hydrazine having the structural formula:

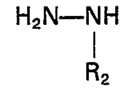

to obtain the compound having the structural formula

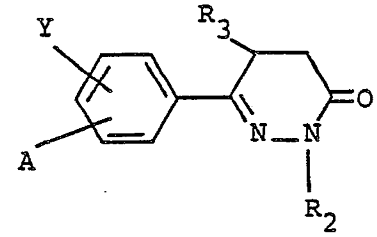

and when desired dehydrogenating to obtain the product

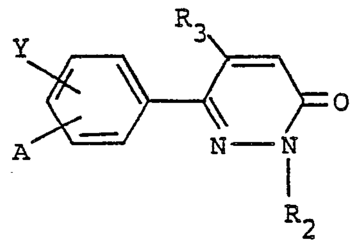

wherein A, Y, $R_2$, and $R_3$ are as defined in Claim 1.

14. A pharmaceutical composition comprising a compound as claimed in any one of Claims 1 to 12, and a pharmaceutically acceptable carrier or diluent therefor.

**Claims for the Contracting State: AT**

1. A process for producing a compound having the following structure:

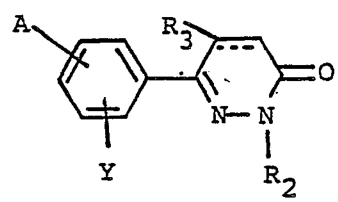

wherein ---- represents a double or single bond between two carbon atoms; $R_2$ and $R_3$ are independently hydrogen or $C_{1-6}$ alkyl; Y is H, halogen, $C_{1-6}$ alkyl, and $C_{1-6}$ alkoxy, and A is any of the groups from a—e, and is attached to the 3- or 4-position of the phenyl ring

a. A =

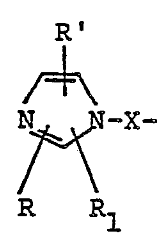

where X is $(CH_2)_n$ or $O(CH_2)_{n+1}$ where n = 1—4; $R_1$, R′, and R are independently hydrogen or $C_{1-6}$ alkyl; hydroxyalkyl, halogen, $CH_2OH$, $SCH_3$, $SOCH_3$, $SO_2CH_3$, $(CH_2)_kNR''R'''$, where k = 0—2 and R″ and R‴ are independently hydrogen or $C_{1-6}$ alkyl, or when attached to the 4- and 5-positions of the imidazole ring may be taken together to form a i) 5-, 6-, or 7-membered ring which may also contain a nitrogen atom; ii) benzene ring which is optionally substituted by halogen, hydroxy, $C_{1-6}$ alkyl, and $C_{1-6}$ alkoxy, and iii) pyridine ring; and the pharmaceutically acceptable salt thereof;
with the proviso that, when X is $(CH_2)_n$, when Y is a hydrogen, when A is attached to the 4-position of the

phenyl ring, and when ----- in the general structural formula represents a single bond, then at least one of R, R', $R_1$, $R_2$ and $R_3$ is other than a hydrogen atom:

b. A =

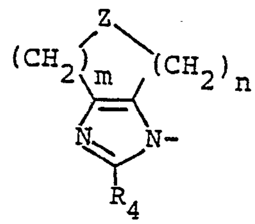

where m + n = 3, $R_4$ is hydrogen or $C_{1-6}$ alkyl; Z is $CH_2$ or $NR_5$ where $R_5$ is hydrogen, $C_{1-6}$ alkyl or $COR_6$ where $R_6$ is $C_{1-6}$ alkyl or aryl and the pharmaceutically acceptable salts thereof

c. A =

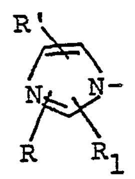

where $R_1$, R', and R are as defined in (a) and is attached to the 3-position of the phenyl ring and the pharmaceutically acceptable salts thereof

d. A =

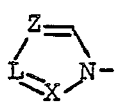

where i) X = L = Z = CH; ii) X = Z = N, and L=CH iii) L = Z = N and X = CH and their pharmaceutically acceptable salts thereof

e. A is same as in I(c) and is attached to the 4-position of the phenyl ring and the pharmaceutically acceptable salts thereof:

which process comprises reacting a substituted -oxo-benzenebutanoic acid having the structural formula:

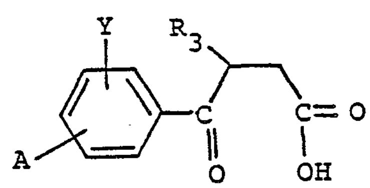

with a substituted hydrazine having the structural formula

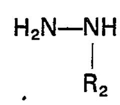

to obtain the compound having the structural formula

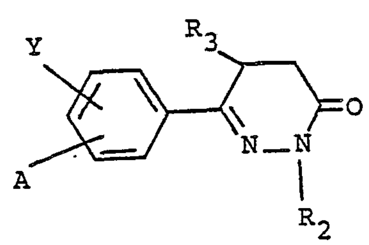

and, when desired, dehydrogenating to obtain the desired product.

2. A process according to Claim 1 for producing compounds having the structure:—

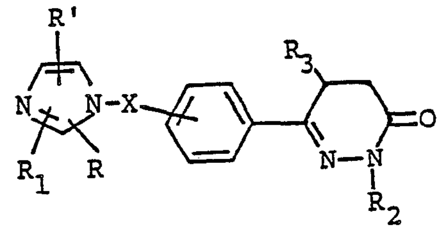

wherein $R_1$, R′, R, X, $R_2$, and $R_3$ are as defined in Claim 1a, and the pharmaceutically acceptable salts thereof.

3. A process according to Claim 1 for producing compounds having the structure:

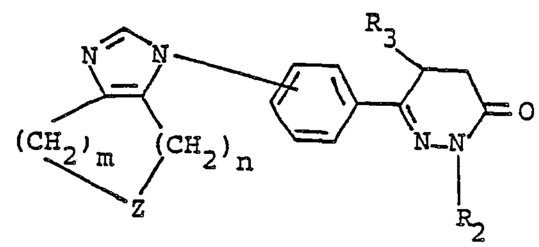

wherein $R_4$, $R_2$, $R_3$, Z, m, and n are as defined in Claim lb, and the pharmaceutically acceptable salts thereof.

4. A process according to Claim 1 for producing compounds having the structure:

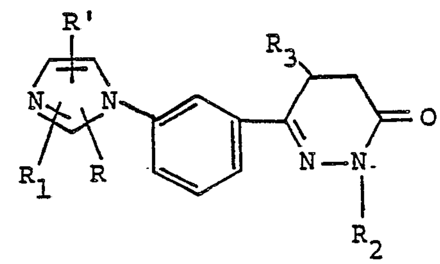

wherein $R_1$, R′, R, $R_2$, and $R_3$ are as defined in Claim 1c, and the pharmaceutically acceptable salts thereof.

5. A process according to Claim 1 for producing compounds having the structure:

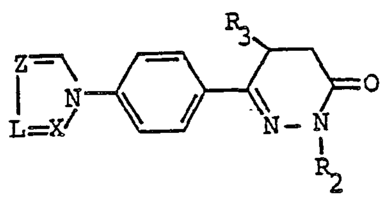

wherein X, L, Z, $R_2$, and $R_3$ are as defined in Claim 1d, and the pharmaceutically acceptable salts thereof.

6. A process according to Claim 1 for producing compounds having the structure:

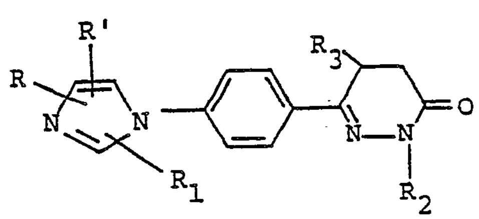

wherein $R_1$, R, R′, $R_2$, and $R_3$ are as defined in Claim 1e and the pharmaceutically acceptable salts thereof.

7. A process according to Claim 1 for producing compounds having the structure:

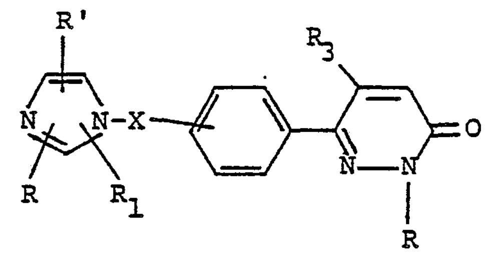

wherein $R_1$, R′, R, X, $R_2$, and $R_3$ are as defined in Claim 1a and the pharmaceutically acceptable salts thereof.

8. A process according to Claim 1 for producing compounds having the structure:

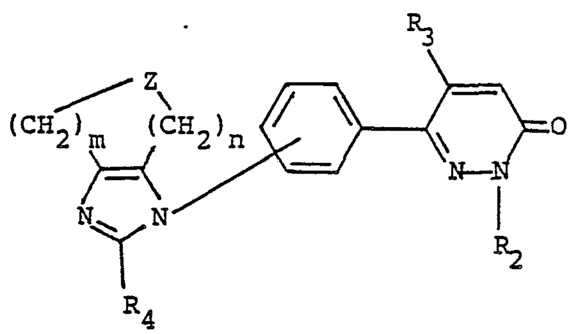

wherein $R_2$, $R_3$, $R_4$, Z, m, and n are as defined in Claim 1b, and the pharmaceutically acceptable salts thereof.

9. A process according to Claim 1 for producing compounds having the structure:

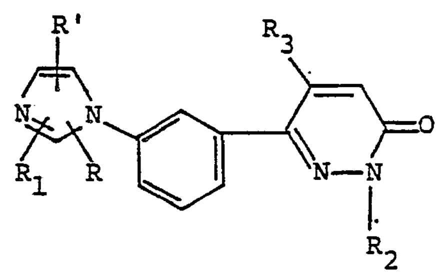

wherein $R_1$, R′, R, $R_2$, and $R_3$ are as defined in Claim 1c, and the pharmaceutically acceptable salts thereof.

10. A process according to Claim 1, for producing compounds having the structure:

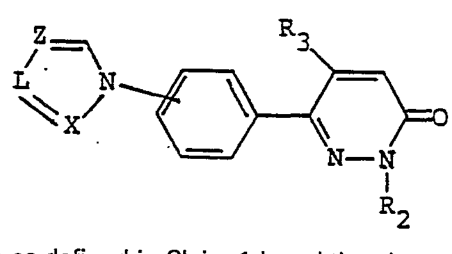

wherein X, L, Z, $R_2$, and $R_3$ are as defined in Claim 1d, and the pharmaceutically acceptable salts thereof.

11. A process according to Claim 1, for producing compounds having the structure:

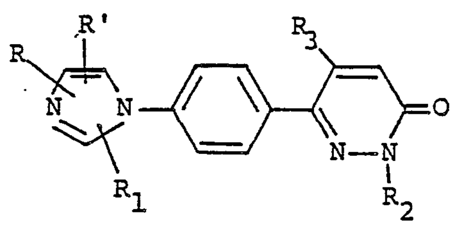

wherein $R_1$, R, R′, $R_2$, and $R_3$ are as defined in Claim 1e and the pharmaceutically acceptable salts thereof.

**Patentansprüche für die Vertragsstaaten: BE CH DE FR GB IT LI LU NL SE**

1. Verbindung mit der Strukturformel:

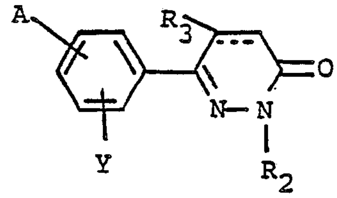

worin ---- eine Doppel- oder Einfachbindung zwischen zwei Kohlenstoffatomen bedeutet; $R_2$ und $R_3$ unabhängig voneinander Wasserstoff oder $C_{1-6}$-Alkyl bedeuten; Y für H, Halogen, $C_{1-6}$-Alkyl und $C_{1-6}$-Alkoxy steht und A irgendeine der Gruppen a—e bedeutet und an der Position 3 oder 4 des Phenylrings hängt,

a. A = 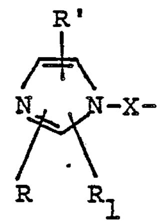

worin X $(CH_2)_n$ oder $O(CH_2)_{n+1}$ bedeutet, wobei n = 1—4; $R_1$, R', und R unabhängig voneinander Wasserstoff oder $C_{1-6}$-Alkyl; Hydroxyalkyl, Halogen, $CH_2OH$, $SCH_3$, $SOCH_3$, $SO_2CH_3$, $(CH_2)_kNR''R'''$ bedeutet, wobei k = 0—2 und R'' and R''' unabhängig voneinander Wasserstoff oder $C_{1-6}$-Alkyl bedeuten oder, wenn sie an den Positionen 4 und 5 des Imidazolrings hängen, zusammengenommen werden, um einen i) 5-, 6-, oder 7-gliedrigen Ring, der auch ein Stickstoffatom enthalten kann, zu bilden; ii) gegebenenfalls durch Halogen, Hydroxy, $C_{1-6}$-Alkyl und $C_{1-6}$-Alkoxy substituierten Benzolring zu bilden, und iii) Pyridinring zu bilden; und das pharmazeutisch akzeptable Salz davon; mit der Maßgabe, daß, wenn X für $(CH_2)_n$ steht, wenn Y Wasserstoff bedeutet, wenn A an der Position 4 des Phenylrings hängt und wenn ---- in der allgemeinen Strukturformel eine Einfachbindung bedeutet, zumindest eines von R, R', $R_1$, $R_2$ und $R_3$ für etwas anderes als Wasserstoff steht:

b. A = 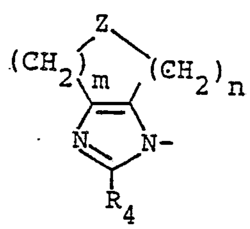

wobei m + n = 3, $R_4$ Wasserstoff oder $C_{1-6}$-Alkyl bedeutet; Z für $CH_2$ oder $NR_5$ steht, wobei $R_5$ Wasserstoff, $C_{1-6}$-Alkyl oder $COR_6$ bedeutet, wobei $R_6$ $C_{1-6}$-Alkyl oder Aryl bedeutet, und die pharmazeutisch akzeptablen Salze davon

c. A = 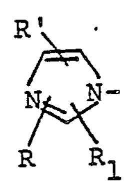

wobei $R_1$, R', und R die in (a) angegebenen Bedeutung haben und dieser Rest A an der Position 3 des Phenylrings hängt und die pharmazeutisch akzeptablen Salze davon

d. A = 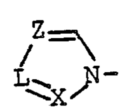

worin i) X = L = Z = CH; ii) X = Z = N und L=CH; iii) L = Z = N und X = CH, und die pharmazeutisch akzeptablen Salze davon

e. A dieselbe Bedeutung hat wie in 1(c) und an der Position 4 des Phenylrings hängt, und die pharmazeutisch akzeptablen Salze davon.

2. Verbindung nach Anspruch 1 mit der Struktur

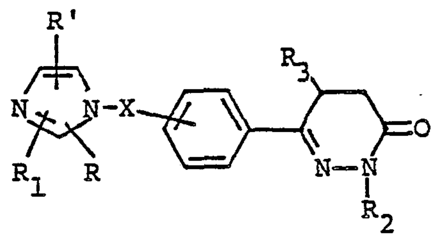

worin $R_1$, R′, R, X, $R_2$ und $R_3$ der in Anspruch 1a angegebenen Bedeutung entsprechen, und deren pharmazeutisch akzeptable Salze.

3. Verbindung nach Anspruch 1 mit der Struktur:

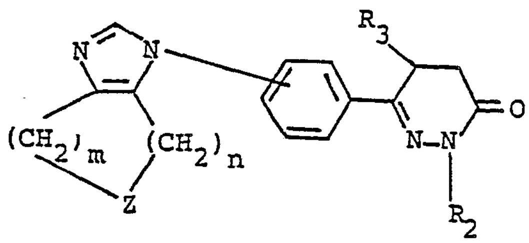

worin $R_4$, $R_2$, $R_3$, Z, m und n der in Anspruch 1b angegebenen Bedeutung entpsrechen, und deren pharmazeutisch akzeptable Salze.

4. Verbindung nach Anspruch 1 mit der Struktur:

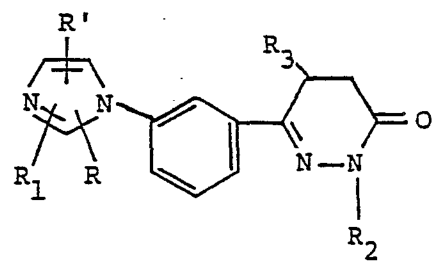

worin $R_1$, R′, R, $R_2$ und $R_3$ der in Anspruch 1c angegebenen Bedeutung entsprechen, und deren pharmazeutisch akzeptable Salze.

5. Verbindung nach Anspruch 1 mit der Struktur:

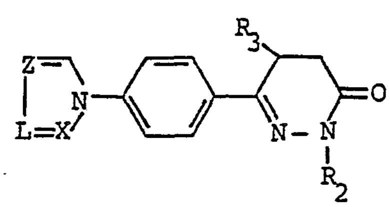

worin X, L, Z, $R_2$ und $R_3$ der in Anspruch 1d angegebenen Bedeutung entsprechen, und deren pharmazeutisch akzeptable Salze.

6. Verbindung nach Anspruch 1 mit der Struktur:

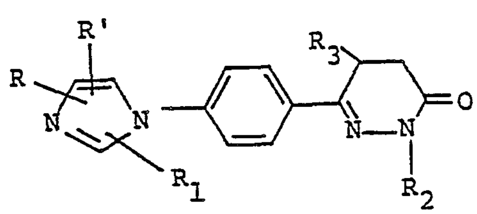

worin $R_1$, R, R', $R_2$ und $R_3$ der in Anspruch 1e angegebenen Bedeutung entsprechen, und deren pharmazeutisch akzeptable Salze.

7. Verbindung nach Anspruch 1 mit der Struktur:

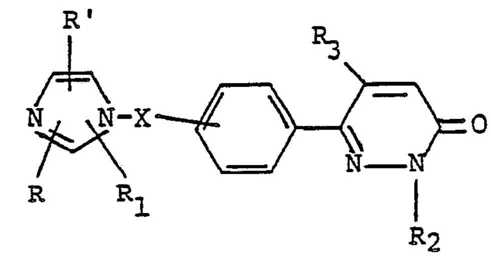

worin $R_1$, R', R, X, $R_2$ und $R_3$ der in Anspruch 1a angegebenen Bedeutung entsprechen, und deren pharmazeutisch akzeptable Salze.

8. Verbindung nach Anspruch 1 mit der Struktur:

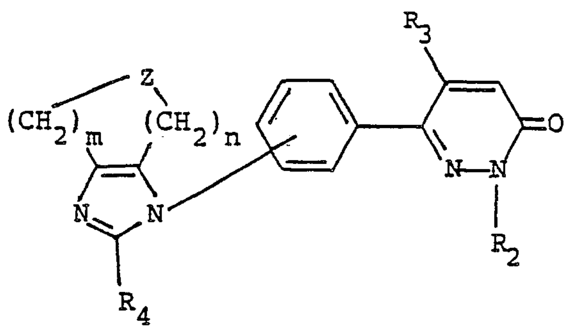

worin $R_2$, $R_3$, $R_4$, Z, m und n der in Anspruch 1b angegebenen Bedeutung entsprechen, und deren pharmazeutisch akzeptable Salze.

9. Verbindung nach Anspruch 1 mit der Struktur:

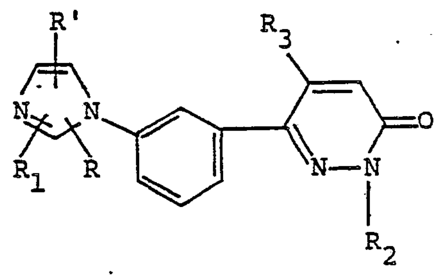

worin $R_1$, R', R, $R_2$ und $R_3$ der in Anspruch 1c angegebenen Bedeutung entsprechen, und deren pharmazeutisch akzeptable Salze.

10. Verbindung nach Anspruch 1 mit der Struktur:

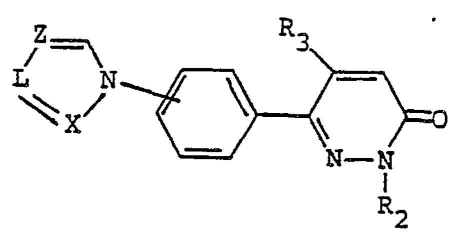

worin X, L, Z, $R_2$ und $R_3$ der in Anspruch 1d angegebenen Bedeutung entsprechen, und deren pharmazeutisch akzeptable Salze.

11. Verbindung nach Anspruch 1 mit der Struktur:

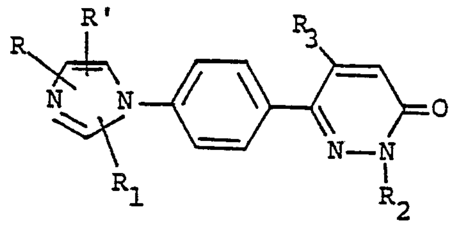

worin $R_1$, R, R', $R_2$ und $R_3$ der in Anspruch 1e angegebenen Bedeutung entsprechen, und deren pharmazeutisch akzeptable Salze.

12. Verbindung nach Anspruch 1, welche
4,5-Dihydro-6-[4-[2-1*H*-imidazol-1-yl)-äthoxy]-phenyl]-3(2*H*)-pyridazinon,
4,5-Dihydro-6-[4-(4,5,6,7-tetrahydro-1*H*-benzimidazol-1-yl)-phenyl]-3(2*H*)-pyridazinon,
4,5-Dihydro-6-[3-(1*H*-imidazol-1-yl)-phenyl]-3-(2*H*)-pyridazinon,
4,5-Dihydro-6-[4-(1*H*-1,2,4-triazol-1-yl)-phenyl]-3(2*H*)-pyridazinon,
4,5-Dihydro-6-[4-(4*H*-1,2,4-triazol-4-yl)-phenyl]-3(2*H*)-pyridazinon,
4,5-Dihydro-6-[4-(1*H*-pyrrol-1-yl)-phenyl]-3(2*H*)-pyridazinon,
4,5-Dihydro-6-[4-(1*H*-imidazol-1-yl)-phenyl]-3(2*H*)-pyridazinon,
4,5-Dihydro-6-[4-(2-methyl-1*H*-imidazol-1-yl)-phenyl]-3(2*H*)-pyridazinon,
4,5-Dihydro-6-[4-(2-äthyl-4-methyl-1*H*-imidazol-1-yl)-phenyl]-3(2*H*)-pyridazinon,
4,5-Dihydro-6-[4-(1*H*-imidazol-1-yl)-phenyl]-5-methyl-3(2*H*)-pyridazinon,
4,5-Dihydro-6-[4-(4,5-diäthyl-1*H*-imidazol-1-yl)-phenyl]-3(2*H*)-pyridazinon,
4,5-Dihydro-6-[4-(1*H*-imidazol-1-yl)-phenyl]-2-methyl-3(2*H*)-pyridazinon,
4,5-Dihydro-6-[4-(1*H*-benzimidazol-1-yl)-phenyl]-3(2*H*)-pyridazinon,
4,5-Dihydro-6-[4-(4-hydroxymethyl-1*H*-imidazol-1-yl)-phenyl-3(2*H*)-pyridazinon,
4,5-Dihydro-6-[4-(2-methylthio-1*H*-imidazol-1-yl)-phenyl]-3(2*H*)-pyridazinon,
4,5-Dihydro-6-[4-(2-methylsulfinyl-1*H*-imidazol-1-yl)-phenyl]-3(2*H*)-pyridazinon,
4,5-Dihydro-6-[4-(2-methylsulfonyl-1*H*-imidazol-1-yl)-phenyl]-3(2*H*)-pyridazinon,
6-[4-[2-(1*H*-Imidazol-1-yl)-äthoxy]-phenyl]-3(2*H*)-pyridazinon,
6-[4-[2-(1*H*-Imidazol-1-yl)-äthyl]-phenyl]-3(2*H*)-pyridazinon,
6-[4-(1*H*-Imidazol-1-yl-methyl)-phenyl]-3(2*H*)-pyridazinon,
6-[4-(4,5,6,7-Tetrahydro-1*H*-benzimidazol-1-yl)-phenyl]-3(2*H*)-pyridazinon,
6-[3-(1*H*-Imidazol-1-yl)-phenyl]-3(2*H*)-pyridazinon,
6-[4-(1*H*-1,2,4-Triazol-1-yl)-phenyl]-3(2*H*)-pyridazinon,
6-[4-(4*H*-1,2,4-Triazol-4-yl)-phenyl]-3(2*H*)-pyridazinon,
6-[4-(1*H*-Pyrrol-1-yl)-phenyl]-3(2*H*)-pyridazinon,
6-[4-(1*H*-Imidazol-1-yl)-phenyl]-3(2*H*)-pyridazinon,
6-[4-(2-Methyl-1*H*-imidazol-1-yl)-phenyl]-3(2*H*)-pyridazinon,
6-[4-(2-Äthyl-4-methyl-1*H*-imidazol-1-yl)-phenyl]-3(2*H*)-pyridazinon,
6-[4-(1*H*-Imidazol-1-yl)-phenyl]-5-methyl-3(2*H*)-pyridazinon,
6-[4-(4,5-Diäthyl-1*H*-imidazol-1-yl)-phenyl]-3(2*H*)-pyridazinon,
6-[4-(4-Hydroxymethyl-1*H*-imidazol-1-yl)-phenyl]-3(2*H*)-pyridazinon,
6-[4-(2-Methylthio-1*H*-imidazol-1-yl)-phenyl]-3(2*H*)-pyridazinon,
6-[4-(2-Methylsulfinyl-1*H*-imidazol-1-yl)-phenyl]-3(2*H*)-pyridazinon,
6-[4-(2-Methylsulfonyl-1*H*-imidazol-1-yl)-phenyl]-3(2*H*)-pyridazinon,
6-[4-(1*H*-Imidazol-1-yl)-phenyl]-2-methyl-3(2*H*)-pyridazinon, oder
6-[4-(1*H*-Benzimidazol-1-yl)-phenyl]-3(2*H*)-pyridazinon ist.

13. Verfahren zur Herstellung von Verbindungen nach Anspruch 1 mit der Strukturformel

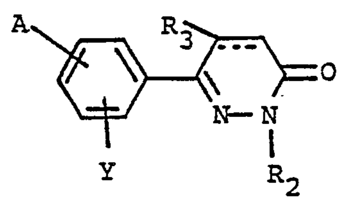

welches die Umsetzung einer substituierten γ-Oxobenzolbutansäure mit der Strukturformel:

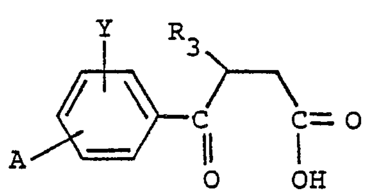

mit einem substituierten Hydrazin mit der Strukturformel:

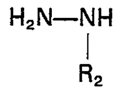

zum Erhalt einer Verbindung mit der Strukturformel

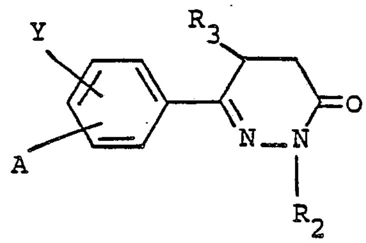

und gewünschtenfalls Dehydrierung zum Erhalt des Produktes

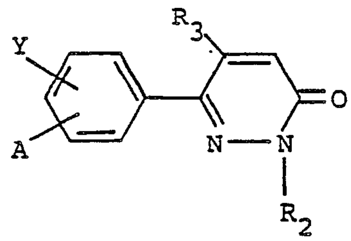

worin A, Y, $R_2$ und $R_3$ der in Anspruch 1 angegebenen Bedeutung entsprechen, umfaßt.

14. Pharmazeutische Zusammensetzung, welche eine Verbindung nach irgendeinem der Ansprüche 1 bis 12 und ein pharmazeutische akzeptables Träger- oder Verdünnungsmittel für diese umfaßt.

**Patentansprüche für den Vertragsstaat: AT**

1. Verfahren zur Herstellung einer Verbindung mit der folgenden Struktur:

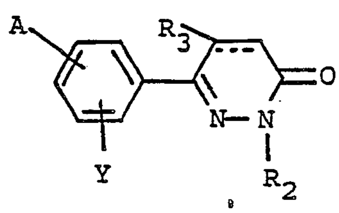

worin ---- eine Doppel- oder Einfachbindung zwischen zwei Kohlenstoffatomen bedeutet, $R_2$ und $R_3$ unabhängig voneinander Wasserstoff oder $C_{1-6}$-Alkyl bedeuten; Y für H, Halogen, $C_{1-6}$-Alkyl und $C_{1-6}$-Alkoxy steht und A eine der Gruppen von a—e bedeutet und an der Position 3 oder 4 des Phenylringes hängt,

a. A =

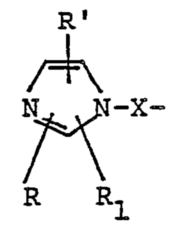

worin X $(CH_2)_n$ oder $O(CH_2)_{n+1}$ steht, wobei n = 1—4; $R_1$, R', und R unabhängig voneinander Wasserstoff oder $C_{1-6}$-Alkyl; Hydroxyalkyl, Halogen, $CH_2OH$, $SCH_3$, $SOCH_3$, $SO_2CH_3$, $(CH_2)_kNR''R'''$ bedeutet, wobei k = 0—2 und R'' and R''' unabhängig voneinander Wasserstoff oder $C_{1-6}$-Alkyl bedeuten oder, wenn sie an den Positionen 4 und 5 des Imidazolrings hängen, zusammengenommen werden können, um einen i) 5-, 6-, oder 7-gliedrigen Ring, der auch ein Stickstoffatom enthalten kann, zu bilden; ii) Benzolring, der gegebenenfalls durch Halogen, Hydroxy, $C_{1-6}$-Alkyl und $C_{1-6}$-Alkoxy substituierten ist, zu bilden; und iii)

Pyridinring zu bilden; und der pharmazeutisch akzeptablen Salze davon; mit der Maßgabe, daß, wenn X für $(CH_2)_n$ steht, wenn Y Wasserstoff bedeutet, wenn A an der Position 4 des Phenylrings hängt und wenn ----- in der allgemeinen Strukturformel eine Einfachbindung bedeutet, zumindest eines von R, R', $R_1$, $R_2$ und $R_3$ für etwas anderes als Wasserstoff steht

b. A = 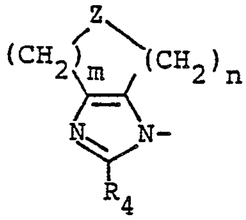

wobei m + n = 3, $R_4$ Wasserstoff oder $C_{1-6}$-Alkyl bedeutet; Z für $CH_2$ oder $NR_5$ steht, worin $R_5$ Wasserstoff, $C_{1-6}$-Alkyl oder $COR_6$ bedeutet, wobei $R_6$ $C_{1-6}$-Alkyl oder Aryl bedeutet, und der pharmazeutisch akzeptablen Salze davon

c. A = 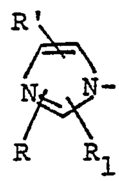

worin $R_1$, R' und R die in (a) angegebenen Bedeutung haben und dieser Rest A an der Position 3 des Phenylrings hängt, und der pharmazeutisch akzeptablen Salze davon

d. A = 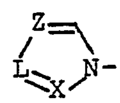

worin i) X = L = Z = CH; ii) X = Z = N und L = CH; iii) L = Z = N und X = CH, und die pharmazeutisch akzeptablen Salze davon

e. A dieselbe Bedeutung hat wie in 1(c) und an der Position 4 des Phenylrings hängt, und der pharmazeutisch akzeptablen Salze davon:

welches Verfahren dadurch gekennzeichnet ist, daß eine substituierte γ-Oxo-Benzolbutansäure mit der Strukturformel

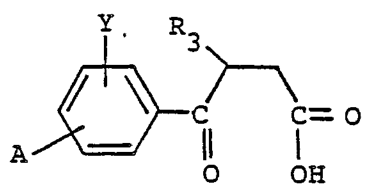

mit einem substituierten Hydrazin mit der Strukturformel:

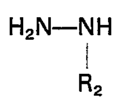

umgesetzt wird, um eine Verbindung mit der Strukturformel

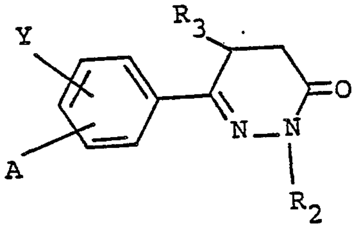

zu erhalten, welche gewünschtenfalls dehydriert wird, um das gewünschte Produkt zu erhalten.

2. Verfahren nach Anspruch 1 zur Herstellung von Verbindungen mit der Struktur:

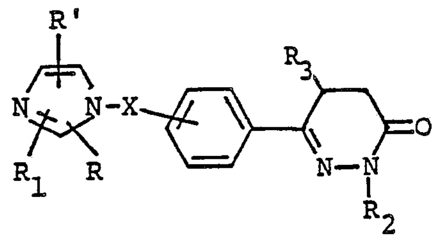

worin $R_1$, R', R, X, $R_2$ und $R_3$ der in Anspruch 1a angegebenen Bedeutung entsprechen, und deren pharmazeutisch akzeptablen Salzen.

3. Verfahren nach Anspruch 1 zur Herstellung von Verbindungen mit der Struktur:

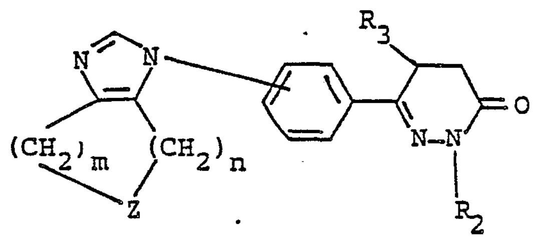

worin $R_4$, $R_2$, $R_3$; Z, m und n der in Anspruch 1b angegebenen Bedeutung entsprechen, und deren pharmazeutisch akzeptablen Salzen.

4. Verfahren nach Anspruch 1 zur Herstellung von Verbindungen mit der Struktur:

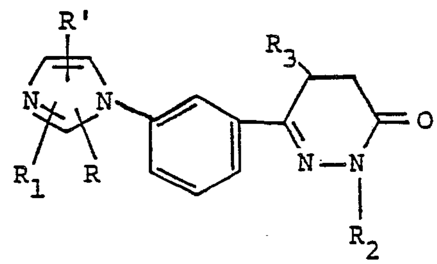

worin $R_1$, R', R, $R_2$ und $R_3$ der in Anspruch 1c angegebenen Bedeutung entsprechen, und deren pharmazeutisch akzeptablen Salzen.

5. Verfahren nach Anspruch 1 zur Herstellung von Verbindungen mit der Struktur:

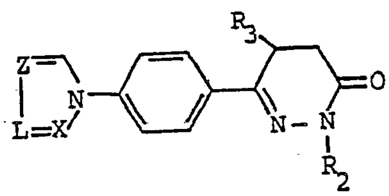

worin X, L, Z, $R_2$ und $R_3$ der in Anspruch 1d angegebenen Definition entsprechen, und deren pharmazeutisch akzeptablen Salzen.

6. Verfahren nach Anspruch 1 zur Herstellung von Verbindungen mit der Struktur:

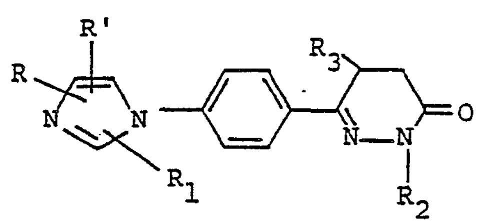

worin $R_1$, R, R', $R_2$ und $R_3$ der in Anspruch 1e angegebenen Bedeutung entsprechen, und deren pharmazeutisch akzeptablen Salzen.

7. Verfahren nach Anspruch 1 zur Herstellung von Verbindungen mit der Struktur:

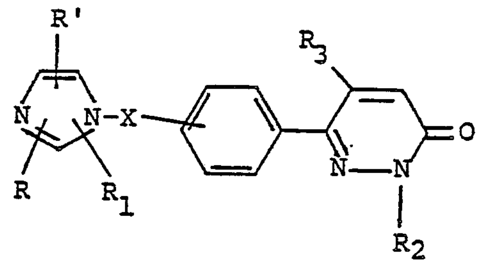

worin $R_1$, R', R, X, $R_2$ und $R_3$ der in Anspruch 1a angegebenen Bedeutung entsprechen, und deren pharmazeutisch akzeptablen Salzen.

8. Verfahren nach Anspruch 1 zur Herstellung von Verbindungen mit der Struktur:

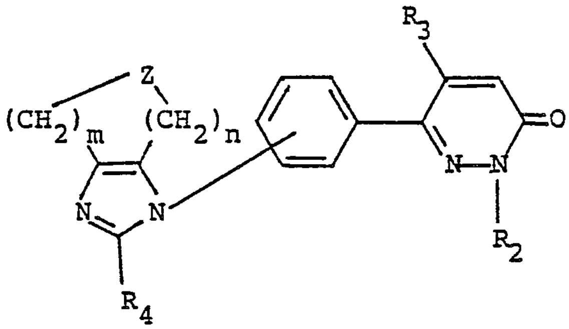

worin $R_2$, $R_3$, $R_4$, Z, m und n der in Anspruch 1b angegebenen Bedeutung entsprechen, und deren pharmazeutisch akzeptablen Salzen.

9. Verfahren nach Anspruch 1 zur Herstellung von Verbindungen mit der Struktur:

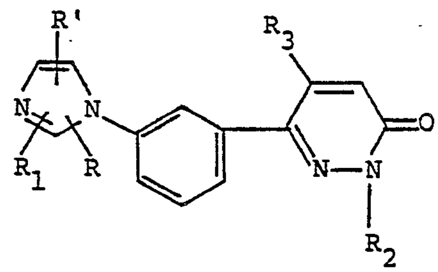

worin $R_1$, R', R, $R_2$ und $R_3$ der in Anspruch 1c angegebenen Bedeutung entsprechen, und deren pharmazeutisch akzeptablen Salzen.

10. Verfahren nach Anspruch 1 zur Herstellung von Verbindungen mit der Struktur:

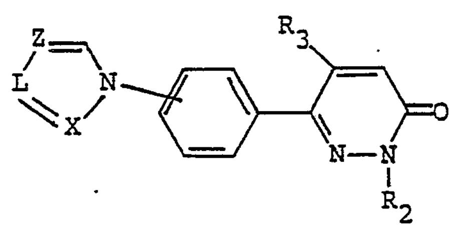

worin X, L, Z, $R_2$ und $R_3$ der in Anspruch 1d angegebenen Bedeutung entsprechen, und deren pharmazeutisch akzeptablen Salzen.

11. Verfahren nach Anspruch 1 zur Herstellung von Verbindungen mit der Struktur:

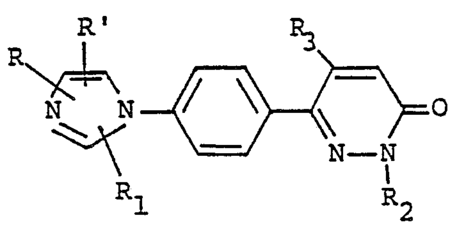

worin $R_1$, R, R', $R_2$ und $R_3$ der in Anspruch 1e angegebenen Bedeutung entsprechen, und deren pharmazeutisch akzeptablen Salzen.

**Revendications pour les Etats contractants: BE CH DE FR GB IT LI LU NL SE**

1. Un composé de formule structurelle:

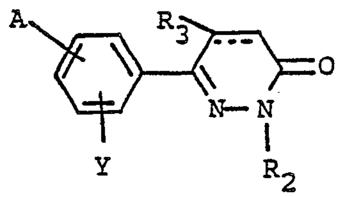

dans laquelle ---- représente une liaison double ou simple entre deux atomes de carbone; $R_2$ et $R_3$ représentent indépendamment un atome d'hydrogène ou un radical alkyle en $C_1$ à $C_6$; Y représente H, un halogène, un radical alkyle en $C_1$ à $C_6$ et un radical alkoxy en $C_1$ à $C_6$; et A représente l'un quelconque des éléments des groupes a—e, et il occupe la position 3 ou la position 4 du cycle phénylique.

a. A = 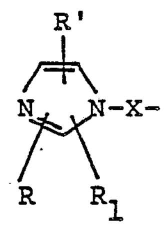

dans lequel: X représente $(CH_2)_n$ ou $O(CH_2)_{n+1}$ dans lequel n = 1 à 4; $R_1$, R', and R représentent indépendamment un atome d'hydrogène, d'halogène ou un radical alkyle en $C_1$ à $C_6$, hydroxyalkyle, $CH_2OH$, $SCH_3$, $SOCH_3$, $SO_2CH_3$, $(CH_2)_kNR''R'''$ dans lequel k = 0 à 2 et R'' et R''' représentent indépendamment un atome d'hydrogène ou un radical alkyle en $C_1$ à $C_6$, ou, lorsqu'ils occupent les positions 4 et 5 du cycle imidazolique, ils peuvent former ensemble i) un cycle à 5, 6 ou 7 éléments pouvant également contenir un atome d'azote; ii) un cycle benzénique éventuellement substitué par un halogène, un hydroxy, une radical alkyle en $C_1$ à $C_6$ et un radical alkoxy en $C_1$ à $C_6$; et iii) un cycle pyridinique;

et leurs sels pharmaceutiquement acceptables;

sous réserve que, lorsque X est $(CH_2)_n$, Y un atome d'hydrogène, A occupe la position 4 du cycle phénylique et ---- de la formule structurelle générale représente une liaison simple, alors un seul d'entre les radicaux R, R', $R_1$, $R_2$ et $R_3$ représente autre chose qu'un atome d'hydrogène;

b. A = 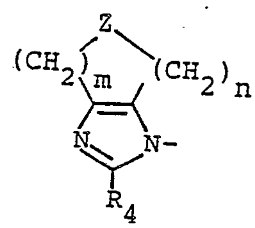

dans lequel: m + n = 3; $R_4$ représente un atome d'hydrogène ou un radical alkyle en $C_1$ à $C_6$; Z représente $CH_2$ ou $NR_5$ dans lequel $R_5$ représente un atome d'hydrogène, un radical alkyle en $C_1$ à $C_6$ ou $COR_6$ dans lequel $R_6$ représente un radical alkyle en $C_1$ à $C_6$ ou un radical aryle; et leurs sels pharmaceutiquement acceptables;

c. A = 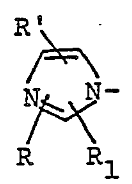

dans lequel $R_1$, R' et R sont tels que définis en (a) et occupent la position 3 du cycle phénylique; et leurs sels pharmaceutiquement acceptables.

d. A = 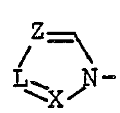

dans lequel: i) X = L = Z = CH; ii) X = Z = N et L = CH; iii) L = Z = N et X = CH; et leurs sels pharmaceutiquement acceptables.

e. A est le même que dans I(c) et il occupe la position 4 dy cycle phénylique; et leurs sels pharmaceutiques acceptables.

2. Composé selon la revendication 1, de structure:

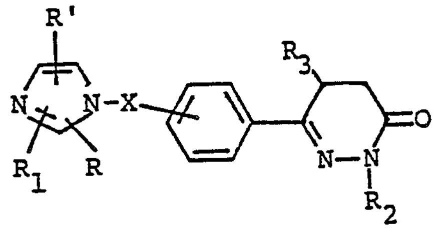

dans laquelle $R_1$, R', R, X, $R_2$ et $R_3$ sont tels que définis dans la revendication Ia, et leurs sels pharmaceutiquement acceptables.

3. Composé selon la revendication 1, de structure:

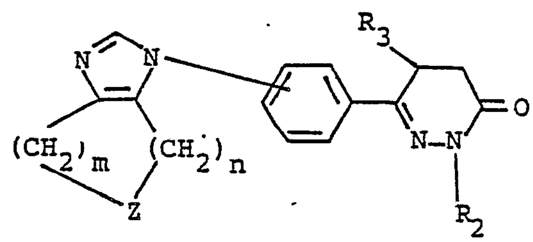

dans laquelle $R_4$, $R_2$, $R_3$, Z, m et n sont tels que définis dans la revendication Ib; et leurs sels pharmaceutiquement acceptables.

4. Composé selon la revendication 1, de structure:

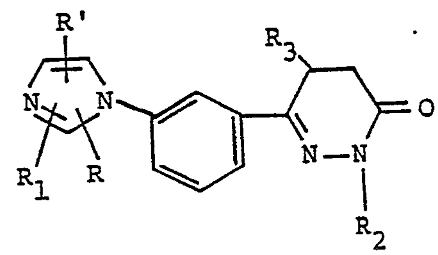

dans laquelle $R_1$, R', R, $R_2$ et $R_3$ sont tels que définis dans la revendication Ic; et leurs sels pharmaceutiquement acceptables.

5. Composé selon la revendication 1, de-structure:

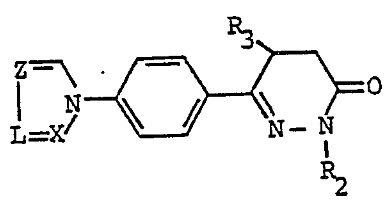

dans laquelle X, L, Z, $R_2$ et $R_3$ sont tels que définis dans la revendication Id; et leurs sels pharmaceutiquement acceptables.

6. Composé selon la revendication 1, de structure:

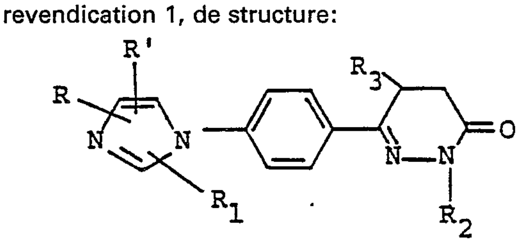

dans laquelle $R_1$, R, R', $R_2$ et $R_3$ sont tels que définis dans la revendication le; et leurs sels pharmaceutiquement acceptables.

7. Composé selon la revendication 1, de structure:

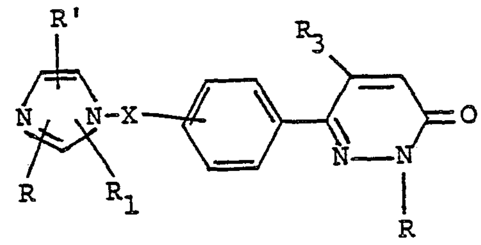

dans laquelle $R_1$, R', R, X, $R_2$ et $R_3$ sont tels que définis dans la revendication la; et leurs sels pharmaceutiquement acceptables.

8. Composé selon la revendication 1, de structure:

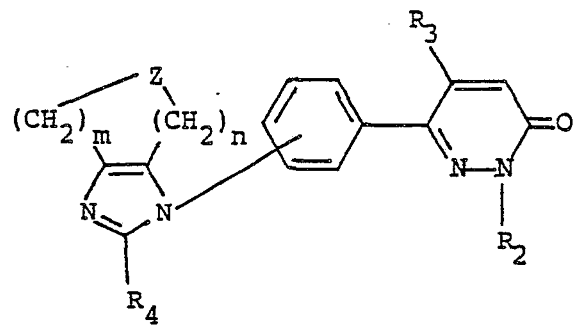

dans laquelle $R_2$, $R_3$, $R_4$, Z, m et n sont tels que définis dans la revendication lb; et leurs sels pharmaceutiquement acceptables.

9. Composé selon la revendication 1, de structure:

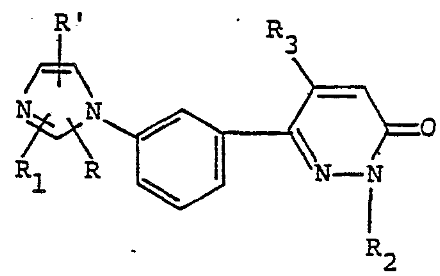

dans laquelle $R_1$, R', R, $R_2$ et $R_3$ sont tels que définis dans la revendication lc; et leurs sels pharmaceutiquement acceptables.

10. Composé selon la revendication 1, de structure:

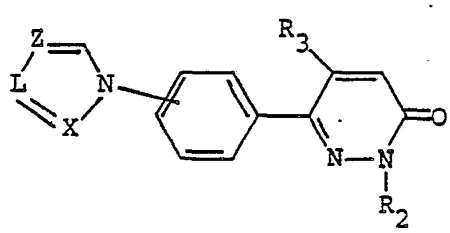

dans laquelle X, L, Z, $R_2$ et $R_3$ sont tels que définis dans la revendication ld; et leurs sels pharmaceutiquement acceptables.

11. Composé selon la revendication 1, de structure:

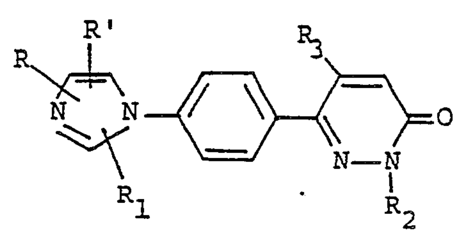

dans laquelle $R_1$, R, R4, $R_2$ et $R_3$ sont tels que définis dans la revendication le; et leurs sels pharmaceutiquement acceptables.

12. Composé selon la revendication 1, qui est la:

4,5-dihydro-6-{4-[2-1*H*-imidazol-1-yl)éthoxy]phényl}-3(2*H*)pyridazinone,
4,5-dihydro-6-[4-(4,5,6,7-tétrahydro-1*H*-benzimidazol-1-yl)-phényl]-3(2*H*)-pyridazinone,
4,5-dihydro-6-[3-(1*H*-imidazol-1-yl)phényl]-3(2*H*)-pyridazinone,
4,5-dihydro-6-[4-(1*H*-1,2,4-triazol-1-yl)phényl]-3(2*H*)-pyridazinone,
4,5-dihydro-6-[4-(4*H*-1,2,4-triazol-4-yl)phényl]-3(2*H*)-pyridazinone,
4,5-dihydro-6-[4-(1*H*-pyrrol-1-yl)phényl]-3(2*H*)-pyridazinone,
4,5-dihydro-6-[4-(1*H*-imidazol-1-yl)phényl]-3(2*H*)-pyridazinone,
4,5-dihydro-6-[4-(2-méthyl-1*H*-imidazol-1-yl)phényl]-3(2*H*)-pyridazinone,
4,5-dihydro-6-[4-(2-éthyl-4-méthyl-1*H*-imidazol-1-yl)phényl]-3(2*H*)-pyridazinone,
4,5-dihydro-6-[4-(1*H*-imidazol-1-yl)phényl]-5-méthyl-3(2*H*)-pyridazinone,
4,5-dihydro-6-[4-(4,5-diéthyl-1*H*-imidazol-1-yl)phényl]-3(2*H*)-pyridazinone,
4,5-dihydro-6-[4-(1*H*-imidazol-1-yl)phényl]-2-méthyl-3(2*H*)-pyridazinone,
4,5-dihydro-6-[4-(1*H*-benzimidazol-1-yl)phényl]-3(2*H*)-pyridazinone,
4,5-dihydro-6-[4-(4-hydroxyméthyl-1*H*-imidazol-1-yl)phényl]-3(2*H*)-pyridazinone,
4,5-dihydro-6-[4-(2-méthylthio-1*H*-imidazol-1-yl)phényl]-3(2*H*)-pyridazinone,
4,5-dihydro-6-[4-(2-méthylsulfinyl-1*H*-imidazol-1-yl)phényl]-3(2*H*)-pyridazinone,
4,5-dihydro-6-[4-(2-méthylsulfonyl-1*H*-imidazol-1-yl)phényl]-3(2*H*)-pyridazinone,
6-{4-(2-1*H*-imidazol-1-yl)éthoxy]phényl}]-3(2*H*)-pyridazinone,
6-{4-[2-(1*H*-imidazol-1-yl)éthyl]phényl}-3(2*H*)-pyridazinone,
6-{4-[(1*H*-imidazol-1-yl-méthyl)phényl}-3(2*H*)-pyridazinone,
6-[4-(4,5,6,7-tétrahydro-1*H*-benzimidazol-1-yl)phényl]-3(2*H*)-pyridazinone,
6-[3-(1*H*-imidazol-1-yl)phényl]-3(2*H*)-pyridazinone,
6-[4-(1*H*-1,2,4-triazol-1-yl)phényl]-3(2*H*)-pyridazinone,
6-[4-(4*H*-1,2,4-triazol-4-yl)phényl]-3(2*H*)-pyridazinone,
6-[4-(1*H*-pyrrol-1-yl)phényl]-3(2*H*)-pyridazinone,
6-[4-(1*H*-imidazol-1-yl)phényl]-3(2*H*)-pyridazinone,
6-[4-(2-méthyl-1*H*-imidazol-1-yl)phényl]-3(2*H*)-pyridazinone,
6-[4-(2-éthyl-4-méthyl-1*H*-imidazol-1-yl)phényl]-3(2*H*)-pyridazinone,
6-[4-(1*H*-imidazol-1-yl)phényl]-5-méthyl-3(2*H*)-pyridazinone,
6-[4-(4,5-diéthyl-1*H*-imidazol-1-yl)phényl]-3(2*H*)-pyridazinone,
6-[4-(4-hydroxyméthyl-1*H*-imidazol-1-yl)phényl]-3(2*H*)-pyridazinone,
6-[4-(2-méthylthio-1*H*-imidazol-1-yl)phényl]-3(2*H*)-pyridazinone,
6-[4-(2-méthylsulfinyl-1*H*-imidazol-1-yl)phényl]-3(2*H*)-pyridazinone,
6-[4-(2-méthylsulfonyl-1*H*-imidazol-1-yl)phényl]-3(2*H*)-pyridazinone,
6-[4-(1*H*-imidazol-1-yl)phényl]-2-méthyl-3(2*H*)-pyridazinone, et
6-[4-(1*H*-benzimidazol-1-yl)phenyl]-3(2*H*)-pyridazinone.

13. Procédé de préparation des composés selon la revendication 1, de formule structurelle:

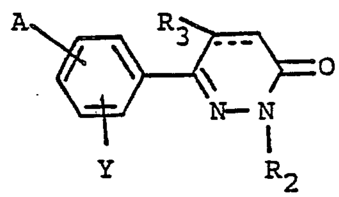

qui consiste à faire réagir un acide γ-oxobenzènebutanoïque substitué de formule structurelle:

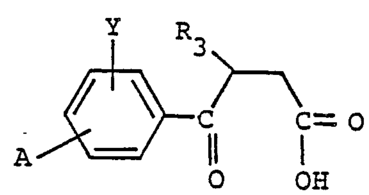

avec une hydrazine substituée de formule structurelle:

$$H_2N—NH(R_2)$$

pour obtenir le composé ayant la formule structurelle:

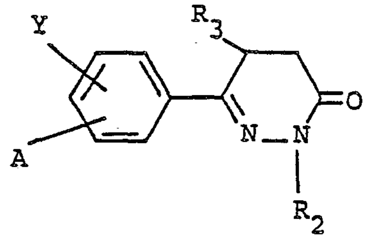

et, lorsqu'on le souhaite, à le déshydrogéner pour obtenir le produit:

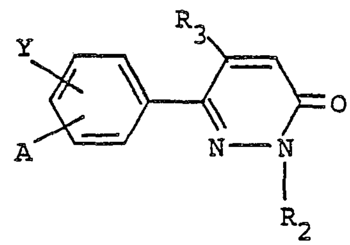

dans lequel A, Y, $R_2$ et $R_3$ sont tels que définis dans la revendication 1.

14. Composition pharmaceutique comprenant un composé selon l'une quelconque des revendications 1 à 12 et son véhicule ou diluant pharmaceutiquement acceptable.

**Revendications pour l'Etat contractant: AT**

1. Procédé de préparation d'un composé de formule de structure suivante:

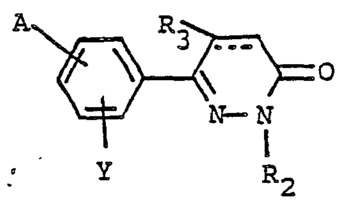

dans laquelle: ---- représente une liaison double ou simple entre deux atomes de carbone; $R_2$ et $R_3$ représentent indépendamment un atome d'hydrogène ou un radical alkyle en $C_1$ à $C_6$; Y représente H, un halogène, un radical alkyle en $C_1$ à $C_6$ et un radical alkoxy en $C_1$ à $C_6$; et A représente l'un quelconque des éléments des groupes a—e, et il occupe la position 3 ou la position 4 du cycle phénylique.

a. A =

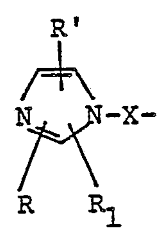

dans lequel: X représente $(CH_2)_n$ ou $O(CH_2)_{n+1}$ dans lequel n = 1—4; $R_1$, R', and R représentent indépendamment un atome d'hydrogène, d'halogène ou un radical alkyle en $C_1$ à $C_6$, hydroxyalkyle, $CH_2OH$, $SCH_3$, $SOCH_3$, $SO_2CH_3$, $(CH_2)_kNR''R'''$ dans lequel k = 0 à 2 et R'' et R''' représentent indépendamment un atome d'hydrogène ou un radical alkyle en $C_1$ à $C_6$, ou, lorsqu'ils occupent les positions 4 et 5 du cycle imidazolique, ils peuvent former ensemble i) un cycle à 5, 6 ou 7 éléments pouvant également contenir un atome d'azote; ii) un cycle benzénique éventuellement substitué par un halogène, un hydroxy, une radical alkyle en $C_1$ à $C_6$ et un radical alkoxy en $C_1$ à $C_6$; et iii) un cycle pyridinique;

et leurs sels pharmaceutiquement acceptables;

sous réserve que, lorsque X est $(CH_2)_n$, Y un atome d'hydrogène, A occupe la position 4 du cycle phénylique et ---- de la formule structurelle générale représente une liaison simple, alors au moins l'un d'entre les radicaux R, R', $R_1$, $R_2$ et $R_3$ représente autre chose qu'un atome d'hydrogène;

b. A = 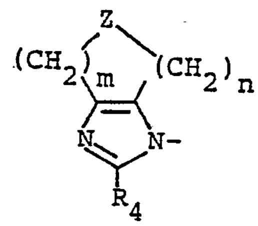

dans lequel: m + n = 3; $R_4$ représente un atome d'hydrogène ou un radical alkyle en $C_1$ à $C_6$; Z représente $CH_2$ ou $NR_5$ dans lequel $R_5$ représente un atome d'hydrogène, un radical alkyle en $C_1$ à $C_6$ ou $COR_6$ dans lequel $R_6$ représente un radical alkyle en $C_1$ à $C_6$ ou un radical aryle; et leurs sels pharmaceutiquement acceptables;

c. A = 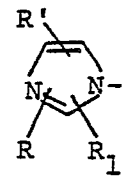

dans lequel $R_1$, R' et R sont tels que définis en (a) et occupent la position 3 du cycle phénylique; et leurs sels pharmaceutiquement âcceptables.

d. A = 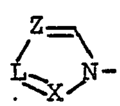

dans lequel: i) X = L = Z = CH; ii) X = Z = N et L = CH; iii) L = Z = N et X = CH; et leurs sels pharmaceutiquement acceptables.

e) A est le même que dans I(c) et il occupe la position 4 du cycle phénylique; et leurs sels pharmaceutiques acceptables.

ce procédé consistant à faire réagir un acide γ-oxobenzènebutanoïque substitué de formule structurelle:

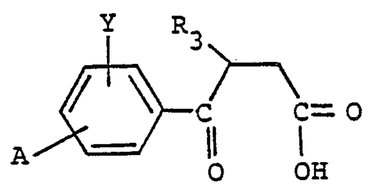

avec une hydrazine substituée de formule structurelle:

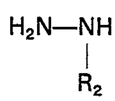

pour obtenir un composé de formule structurelle:

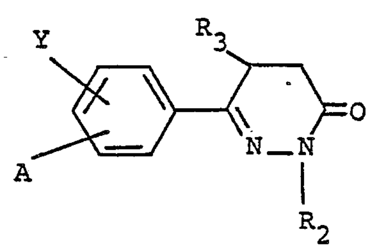

et, lorsqu'on le souhaite, à le déshydrogéner pour obtenir le produit recherché.

2. Procédé selon la revendication 1 de préparation de composés de structure:

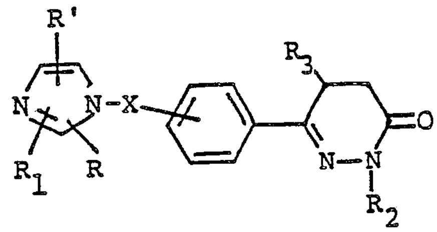

dans laquelle $R_1$, R′, R, X, $R_2$ et $R_3$ sont tels que définis dans la revendication 1a; et leurs sels pharmaceutiquement acceptables.

3. Procédé selon la revendication 1 de préparation de composés de structure:

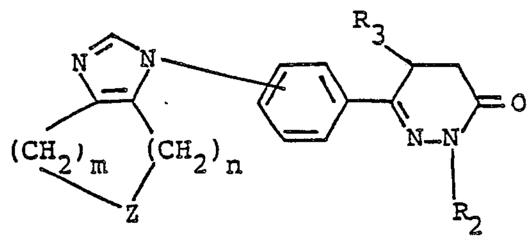

dans laquelle $R_4$, $R_2$, $R_3$, Z, m et n sont tels que définis dans la revendication Ib; et leurs sels pharmaceutiquement acceptables.

4. Procédé selon la revendication 1 de préparation de composés de structure:

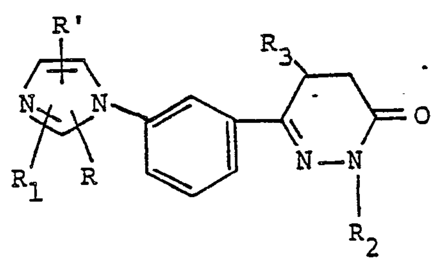

dans laquelle $R_1$, R′, R, $R_2$ et $R_3$ sont tels que définis dans la revendication Ic; et leurs sels pharmaceutiquement acceptables.

5. Procédé selon la revendication 1 de préparation de composés de structure:

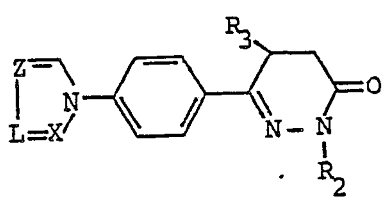

dans laquelle X, L, Z, $R_2$ et $R_3$ sont tels que définis dans la revendication Id; et leurs sels pharmaceutiquement acceptables.

6. Procédé selon la revendication 1 de préparation de composés de structure:

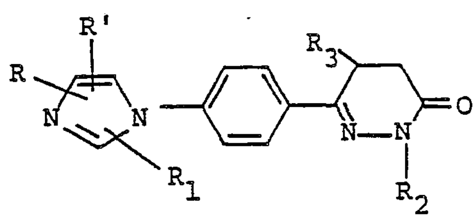

dans laquelle $R_1$, R, R′, $R_2$ et $R_3$ sont tels que définis dans la revendication Ie; et leurs sels pharmaceutiquement acceptables.

7. Procédé selon la revendication 1 de préparation de composés de structure:

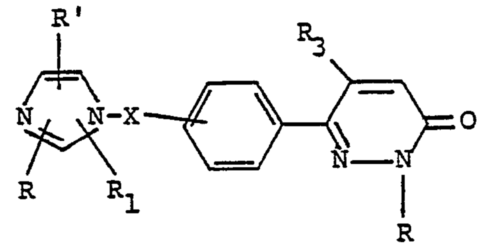

dans laquelle $R_1$, R', R, X, $R_2$ et $R_3$ sont tels que définis dans la revendication Ia; et leurs sels pharmaceutiquement acceptables.

8. Procédé selon la revendication 1 de préparation de composés de structure:

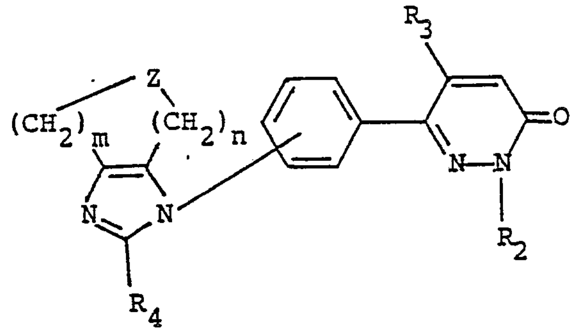

dans laquelle $R_2$, $R_3$, $R_4$, Z, m et n sont tels que définis dans la revendication Ib; et leurs sels pharmaceutiquement acceptables.

9. Procédé selon la revendication 1 de préparation de composés de structure:

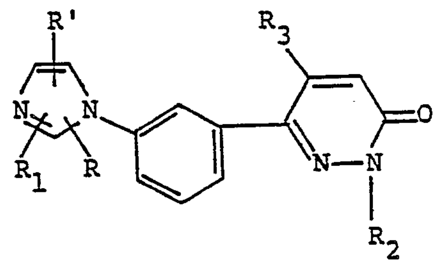

dans laquelle $R_1$, R', R, $R_2$ et $R_3$ sont tels que définis dans la revendication Ic; et leurs sels pharmaceutiquement acceptables.

10. Procédé selon la revendication 1 de préparation de composés de structure:

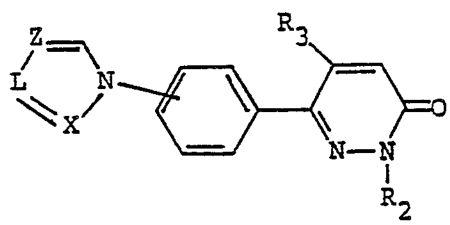

dans laquelle X, L, Z, $R_2$ et $R_3$ sont tels que définis dans la revendication Id; et leurs sels pharmaceutiquement acceptables.

11. Procédé selon la revendication 1 de préparation de composés de structure:

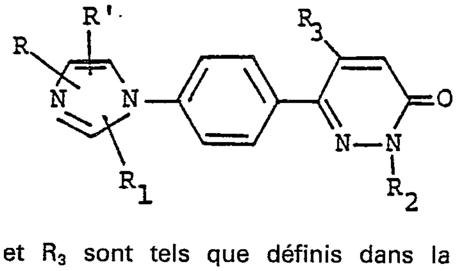

dans laquelle $R_1$, R, R4, $R_2$ et $R_3$ sont tels que définis dans la revendication Ie; et leurs sels pharmaceutiquement acceptables.